# EUROPEAN PATENT SPECIFICATION

(11) **EP 3 661 526 B1**
(45) Date of publication and mention of the grant of the patent: **12.07.2023**
(21) Application number: 18842075.6
(22) Date of filing: 02.08.2018
(51) Int. Cl.: A61K 35/12, A61K 35/34, A61K 35/545, A61K 48/00, C12N 5/095, G01N 33/574, A61P 35/00

(54) **CARDIOSPHERE-DERIVED CELLS AND THEIR EXTRACELLULAR VESICLES FOR TREATMENT AND PREVENTION OF CANCER**
AUS KARDIOSPHÄREN ABGELEITETE ZELLEN UND DEREN EXTRAZELLULÄRE VESIKEL ZUR BEHANDLUNG UND VORBEUGUNG VON KREBS
CELLULES DÉRIVÉES DE CARDIOSPHÈRE ET LEURS VÉSICULES EXTRACELLULAIRES POUR LE TRAITEMENT ET LA PRÉVENTION DU CANCER

(30) Priority: 04.08.2017 US 201762541584 P
(43) Date of publication of application: 10.06.2020
(60) Divisional application: 23177120.5
(73) Proprietor: Cedars-Sinai Medical Center, Los Angeles, CA 90048 (US)
(72) Inventor: MARBAN, Eduardo, Los Angeles California 90048 (US); GRIGORIAN, Lilian, Los Angeles California 90048 (US)
(74) Representative: Forrest, Stuart
(86) International application number: PCT/US2018/044956
(87) International publication number: WO 2019/028223

(56) References cited:
- WO-A1-2015/085096
- WO-A1-2015/120150
- WO-A1-2016/054569
- WO-A1-2016/054591
- WO-A1-2016/057560
- WO-A2-2014/028493
- GRIGORIAN-SHAMAGIAN et al.: "Harnessing the heart's resistance to malignant tumors: cardiac-derived extracellular vesicles decrease fibrosarcoma growth and leukemia-related mortality in rodents", Oncotarget, vol. 8, no. 59, 24 August 2017 (2017-08-24), pages 99624-99636, XP055571409,
- MIDDLETON et al.: "Newt cells secrete extracellular vesicles with therapeutic bioactivity in mammalian cardiomyocytes", J Extracell Vesicles, vol. 7, no. 1, 15 April 2018 (2018-04-15), pages 1-15, XP055571415,

## Description

### BACKGROUND

### Field

Described herein are methods and compositions related to cardiospheres, cardiosphere-derived cells (CDCs), and extracellular vesicles (EVs) (including but not limited to exosomes (XOs) and microvesicles (MVs)) for treatment and prevention of cancer.

### Background

Cells release into the extracellular environment diverse types of membrane vesicles of endosomal and plasma membrane origin called XOs and MVs, respectively. These extracellular vesicles represent a mode of intercellular communication by serving as vehicles for transfer between cells of membrane and cytosolic proteins, lipids, and RNA.

WO2016057560A1 discloses compositions and techniques related to generation and therapeutic application of cell-derived exosomes for modulating inflammation and/or conferring cardioprotection to a subject suffering from an inflammatory related disease and/or condition.

WO2016054591A1 discloses compositions and techniques related to generation and therapeutic application of cell-derived exosomes isolated from cardiosphere-derived cells (CDCs) for treatment of heart failure secondary to a chronic degenerative muscular disease.

WO2016054569A1 discloses compositions and techniques related to generation and therapeutic application of compositions including CDCs or stem cells, progenitors and/or precursors compositions, for treatment of heart related diseases and/or conditions such as pulmonary hypertension or pulmonary arterial hypertension.

WO2015120150A1 discloses purified exosomes which induce apoptosis in breast cancer cells and which are derived from a cultured medium of histologically normal breast tissue cells that are obtained from tumor-adjacent normal breast tissue, and related methods and pharmaceutical formulations for treating breast cancer.

### SUMMARY

The invention is defined by the claims. Any subject-matter which falls outside the scope of the claims is presented for comparative purposes only.

According to the invention there is provided extracellular vesicles derived from cardiosphere-derived cells (CDC-EVs) for use in a method of treating or preventing cancer in a subject in need thereof, the method comprising i) administering to the subject a therapeutically effective amount of extracellular vesicles (CDC-EVs); or ii) administering a composition comprising CDC-EVs to a subject who is susceptible to cancer, wherein the subject possesses one or more genetic mutations that make the subject susceptible to cancer.

Also disclosed herein are methods of treating cancer. In the disclosure, the methods include administering a therapeutically effective amount of a composition including EV s, CDCs, and/or cardiospheres to a subject afflicted with cancer, thereby treating the subject. In some embodiments, the administration is systemic. In some embodiments, the systemic administration is intraperitoneal injection. In some embodiments, the administration is local. In some embodiments, the local administration is subcutaneous. In some embodiments, the EVs are obtained from cardiospheres, cardiosphere-derived cells (CDCs) or a newt Al cell line. In the disclosure, treating cancer in the subject includes a reduction in tumor weight. In the disclosure, treating cancer in the subject includes a reduction in tumor vascularization. In the disclosure, treating cancer in the subject includes a reduction in tumor invasion, metastasis, or both. In the disclosure, treating cancer in the subject includes a decrease in serum lactate dehydrogenase.

Also disclosed herein is a method of preventing cancer, including administering a composition including EVs, CDCs, and/or cardiospheres to a subject susceptible to cancer. In the disclosure, the subject possess one or more genetic mutations. In the disclosure, the administration is systemic. In the disclosure, the systemic administration is intraperitoneal injection. In the disclosure, the administration is local. In the disclosure, the local administration is subcutaneous. In the disclosure, the extracellular vesicles are obtained from cardiospheres, CDCs or newt A1 cell line.

Some disclosures pertain to method of treating cancer in a subject in need thereof, the method comprising administering to the subject a therapeutically effective amount of cardiosphere derived cells (CDCs) and/or a therapeutically effective amount of extracellular vesicles (EVs). In the disclosure , the EVs comprise one or more of MVs, XOs, CDC-derived extracellular vesicles (CDC-EVs), CDC-derived microvesicles (CDC-MVs), CDC-derived exosomes (CDC-XOs), or combinations thereof.

In the disclosure, the therapeutically effective amount of CDCs and/or EVs is administered to the subject systemically. In the disclosure , the systemic administration of said therapeutically effective amount of CDCs and/or EVs is via intravenous injection. In the disclosure , the systemic administration of said therapeutically effective amount of CDCs and/or CDC-EVs is via intraperitoneal injection. In the disclosure, the systemic administration of said therapeutically effective amount of CDCs and/or CDC-EVs is via injection into the right ventricle or left ventricle of the heart.

In the disclosure, the administration is local. In the disclosure the local administration is subcutaneous.

In the disclosure, the administration of a therapeutically effective amount of CDCs and/or EVs is via two or more injections. In the disclosure, the administration of a therapeutically effective amount of CDCs and/or EVs is via a single administration.

In the disclosure, the CDCs and/or EVs are allogeneic, and the subject is a human subject. In the disclosure , the EVs are isolated from CDCs grown in serum-free media. In the disclosure, the CDCs or EVs are provided as a pharmaceutical composition comprising a pharmaceutically acceptable carrier. In the disclosure, the EVs are obtained from cardiospheres, CDCs, or a newt A1 cell line.

In the disclosure, the treatment comprises one or more of a reduction in tumor weight, a reduction in tumor vascularization, a reduction in tumor invasion, metastasis, or combinations thereof.

Some disclosures pertain to a method of preventing, mitigating, reversing, or treating cancer in a subject in need thereof. In the disclosure , the method comprising administering to the subject a prophylactically or therapeutically effective amount of extracellular vesicles (EVs), wherein the EVs are obtained from cardiospheres, cardiosphere-derived cells (CDCs), or newt A1 cell line.

Some disclosures pertain to a formulation of EVs for use in treating cancer in a subject in need thereof, wherein said EVs are obtained from cardiospheres, CDCs, or newt A1 cell line.

Any of the disclosures described above, or described elsewhere herein, can include one or more of the following features.

In the disclosure , the EVs, CDCs, or newt A1 cell line are derived from regenerative stem cells such as embryonic stem cells, pluripotent stem cells, multipotent stem cells, induced pluripotent stem cells, post-natal stem cells, adult stem cells, mesenchymal stem cells, hematopoietic stem cells, endothelial stem cells, epithelial stem cells, neural stem cells, cardiac stem cells including cardiac progenitor cells, bone marrow-derived stem cells, adipose-derived stem cells, hepatic stem cells, peripheral blood derived stem cells, cord blood-derived stem cells, or placental stem cells.

In the disclosure, the extracellular vesicles are XOs, MVs, membrane particles, membrane vesicles, exosome-like vesicles, ectosomes, ectosome-like vesicles, or exovesicles.

In the disclosure, the extracellular vesicles are obtained from CDCs.

In the disclosure, the subject is a mammal. In the disclosure , the subject is a human.

Various disclosures provided for herein include treatment or prevention of the following non-limiting examples of cancers including, but not limited to, acute lymphoblastic leukemia (ALL), acute myeloid leukemia (AML), adrenocortical carcinoma, Kaposi sarcoma, lymphoma, gastrointestinal cancer, appendix cancer, central nervous system cancer, basal cell carcinoma, bile duct cancer, bladder cancer, bone cancer, brain tumors (including but not limited to astrocytomas, spinal cord tumors, brain stem glioma, craniopharyngioma, ependymoblastoma, ependymoma, medulloblastoma, medulloepithelioma), breast cancer, bronchial tumors, Burkitt lymphoma, cervical cancer, colon cancer, chronic lymphocytic leukemia (CLL), chronic myelogenous leukemia (CML), chronic myeloproliferative disorders, ductal carcinoma, endometrial cancer, esophageal cancer, gastric cancer, Hodgkin lymphoma, non-Hodgkin lymphoma, hairy cell leukemia, renal cell cancer, leukemia, oral cancer, nasopharyngeal cancer, liver cancer, lung cancer (including but not limited to, non-small cell lung cancer (NSCLC) and small cell lung cancer), pancreatic cancer, bowel cancer, lymphoma, melanoma, ocular cancer, ovarian cancer, pancreatic cancer, prostate cancer, pituitary cancer, uterine cancer, and vaginal cancer. In the disclosure, the cancer is selected from one or more of acute lymphoblastic leukemia (ALL), acute myeloid leukemia (AML), chronic lymphocytic leukemia (CLL), chronic myelogenous leukemia (CML), acute myelogenous leukemia, acute myeloid leukemia, or hairy cell leukemia. In the disclosure, the cancer is selected from one or more of rhabdomyosarcoma, vascular tumors, Ewing Sarcoma, Kaposi Sarcoma, osteosarcoma (Bone Cancer), soft tissue sarcoma, or uterine sarcoma. In the disclosure, the cancer is a form of leukemia. In the disclosure, the cancer is a sarcoma. In the disclosure , the method includes treating a subject suffering from leukemia. In the disclosure , the method includes treating a subject having a sarcoma.

In the disclosure, the EVs are obtained from CDCs using 1000kDa-10kDa process. In the disclosure, the 1000kDa-10kDa process comprises sequentially using a 1000kDa filter for ultra-filtration, and then a 10kDa for diafiltration.

Some disclosures pertain to a method of treating cancer in a subject in need thereof, the method comprising administering to the subject at least two doses of a therapeutically effective amount of CDCs or EVs. In the disclosure , the at least two doses are administered about 1 to 2 weeks apart from each other. In the disclosure , the two doses are configured to not induce a significant immune response in the subject. In the disclosure , the doses are given to the patient via systemic administration, via local administration, or both. In the disclosure, the administration comprises intravenous injection. In the disclosure, the CDCs are allogeneic human CDCs, and the subject is a human.

Some disclosures pertain to a method of treating cancer in a subject in need thereof, the method comprising administering a therapeutically effective amount of a composition comprising EVs to a subject afflicted with cancer, thereby treating the subject. In the disclosure , the administration is systemic. In the disclosure , the systemic administration is via intraperitoneal injection. In the disclosure , the administration is local. In the disclosure, the local administration is subcutaneous. In the disclosure , the EVs are obtained from cardiospheres, CDCs, or newt A1 cell line. In the disclosure , the treating of cancer in the subject comprises a reduction in tumor weight. In the disclosure , the treating of cancer in the subject comprises a reduction in tumor vascularization. In the disclosure , the treating of cancer in the subject comprises a reduction in tumor invasion, metastasis, or both. In the disclosure , the treating of cancer in the subject comprises a decrease in serum lactate dehydrogenase.

Some disclosures pertain to a method of preventing cancer in a patient. In some disclosures , the method comprises administering a composition comprising EVs to a subject who is susceptible to cancer. In the disclosure , the subject possesses one or more genetic mutations that make the subject susceptible to cancer. In the disclosure , the administration is systemic. In the disclosure, the systemic administration is intraperitoneal injection. In the disclosure , the administration is local. In the disclosure , the local administration is subcutaneous. In the disclosure , the EVs are obtained from cardiospheres, CDCs or newt A1 cell line.

Some disclosures pertain to the use of a composition comprising a therapeutically effective amount of CDCs and/or a therapeutically effective amount of EVs for treating cancer. In the disclosure , the composition is suitable for administration to a subject having cancer or at risk for cancer, and wherein the administration of the composition treats and/or prevents said cancer. In the disclosure , the EVs comprise one or more of MVs, XOs, CDC--EVs, CDC-MVs, CDC-XOs, or combinations thereof.

Any of the disclosures described above, or described elsewhere herein, can include one or more of the following features.

In the disclosure , the EV is an XO, MV, membrane particle, membrane vesicle, exosome-like vesicle, ectosome, ectosome-like vesicle, exovesicle, epididimosome, argosome, promininosome, prostasome, dexosome, texosome, archeosome, oncosome, or the like.

In the disclosure , the subject for treatment is a mammal. In some embodiments, the subject is a human. In the disclosure , the human is a patient suffering from cancer and/or at risk for developing cancer.

In the disclosure, said extracellular vesicles are derived from CDCs cultured in serum-free medium (e.g., IMDM) and incubated at about 5% O₂ for about 15 days, wherein said extracellular vesicles are obtained after filtering CDC condition medium containing extracellular vesicles through a 3-1000 kDa (e.g., 10 kDa) pore sized filter. More generally, according to the disclosure , said extracellular vesicles are derived from CDCs cultured in serum-containing or serum-free medium and incubated at 2-20% O₂ for 1-15 days, wherein said extracellular vesicles are obtained after filtering CDC condition medium containing extracellular vesicles through a 3-1000 kDa (e.g., 10 kDa) pore sized filter. Alternatively, extracellular vesicles are obtained by precipitation with polyethylene glycol (e.g., 25% PEG).

In the disclosure , EVs are formulated in a crystalloid solution (e.g., Plasmalyte, normal saline), aqueous solution, gel, ointment, cream, topical or implantable hydrogel, powder, sustained-release polymer (e.g., PLGA and PLA), polyethylene glycol (PEG)-containing solution, suspension, emulsion, as part of a drug delivery device, insert, patch, or the like. In the disclosure , prior to use, EVs are resuspended in an appropriate buffer, e.g., sterile PBS with or without human serum albumin. In the disclosure , EVs can be stored for future use, e.g., frozen at -80°C.

In the disclosure , EVs are derived from human or animal cells. In the disclosure , EVs are prepared from cardiospheres or CDCs. In the disclosure , EVs are prepared from regenerative stem cells such as embryonic stem cells, pluripotent stem cells, multipotent stem cells, induced pluripotent stem cells, post-natal stem cells, adult stem cells, mesenchymal stem cells, hematopoietic stem cells, endothelial stem cells, epithelial stem cells, neural stem cells, cardiac stem cells including cardiac progenitor cells, bone marrow-derived stem cells, adipose-derived stem cells, hepatic stem cells, peripheral blood derived stem cells, cord blood-derived stem cells, placental stem cells, or the like.

In the disclosure , EVs are modified (e.g., genetically or otherwise) to direct them to a specific target site. For example, a modification may, in the disclosure , comprise inducing expression of a specific cell-surface marker on the exosome, which results in specific interaction with a receptor on a desired target tissue. In a disclosure , the native contents of the exosome are removed and replaced with, or supplemented with, desired exogenous proteins and/or nucleic acids.

In the disclosure , EVs include one or more microRNAs selected from: miR-146a, miR-148a, miR-22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and miR-23a. In the disclosure , EVs (e.g., XOs and/or MVs) comprise miR-146a and miR-210. In the disclosure , EVs include one or more microRNAs selected from: hsa-miR-23a-3p, hsa-miR-130a-3p, hsa-miR-21-5p, hsa-miR-4516, hsa-let-7a-5p, hsa-miR-125b-5p, hsa-miR-199a-3p, hsa-miR-199b-3p, hsa-miR-22-3p, hsa-miR-24-3p, hsa-miR-1290, hsa-miR-320e, hsa-miR-423-5p, hsa-miR-22-3p, hsa-miR-222-3p (also known as miR-221-3p), hsa-miR-100-5p, hsa-miR-337-5p, hsa-miR-27b-3p, hsa-miR-1915-3p, and hsa-miR-29b-3p, hsa-miR-25-3p (also known as miR-92a-3p).

In the disclosure , EVs contain biological proteins, e.g., transcription factors, cytokines, growth factors, and similar proteins capable of modulating signaling pathways in a target cell. In the disclosure , the biological protein is capable of facilitating regeneration and/or improved function of a tissue. In the disclosure , the biological protein is capable of modulating pathways related to Irak1, Traf6, toll-like receptor (TLR) signaling pathway, NOX-4, SMAD-4, and/or TGF-β. In the disclosure , the biological protein is related to exosome formation and packaging of cytosolic proteins such as Hsp70, Hsp90, 14-3-3 epsilon, PKM2, GW182 and AGO2. In the disclosure , EVs (e.g., XOs and/or MVs) contain signaling lipids, e.g., ceramide and derivatives.

In the disclosure, the EVs express tetraspanins, e.g., CD63, CD81, CD82, CD53, and/or CD37. In the disclosure , EVs (e.g., XOs and/or MVs) express one or more lipid raft associated proteins (e.g., glycosylphosphatidylinositol-anchored proteins and flotillin), cholesterol, sphingomyelin, and/or hexosylceramides.

In the disclosure , the EVs have a diameter of, *e.g.,* about 15-250 nm, about 15-205 nm, about 90-220 nm, about 30-200 nm, about 20-150 nm, about 70-150 nm, or about 40-100 nm. In the disclosure, the EVs have a diameter of, *e.g.,* about 100-1000 nm.

In the disclosure , the EVs are purified such that contaminants or undesired compounds are removed from the XOs. In the disclosure, the patient is administered substantially purified XOs such that about 50% to 90%, or up to 100%, of the contaminants are removed from the XOs. In the disclosure , an exosome preparation is essentially free of non-exosome components.

In the disclosure , the EVs are administered in combination with one or more additional agents. For example, in several disclosures , the XOs are administered in combination with one or more proteins or nucleic acids derived from the exosome. In several disclosures , the cells from which the XOs are isolated are administered in conjunction with the XOs. In several disclosures , such an approach advantageously provides an acute and more prolonged duration of exosome delivery (e.g., acute based on the actual exosome delivery and prolonged based on the cellular delivery, the cells continuing to secrete XOs post-delivery).

In the disclosure , the dose of EVs ranges about 1.0 × 10⁵ to about 1.0 × 10¹³ XOs. In certain disclosures , the exosome dose is administered on a per kilogram basis, e.g., about 1.0 × 10⁵ XOs/kg to about 1.0 × 10⁹ XOs/kg. In additional disclosures , XOs are delivered in an amount based on the mass of the target tissue, e.g., about 1.0 × 10⁵ XOs/gram of target tissue to about 1.0 × 10⁹ XOs/gram of target tissue. In the disclosure, XOs are administered based on a ratio of the number of XOs to the number of cells in a particular target tissue. If XOs are to be administered in conjunction with the concurrent therapy (*e.g.,* cells that can still shed XOs, pharmaceutical therapy, nucleic acid therapy, and the like) the dose of XOs administered can be adjusted accordingly (e.g., increased or decreased as needed to achieve the desired therapeutic effect).

Some disclosures provide a formulation comprising EVs (e.g., XOs and/or MVs) for use in the prevention or treatment of cancer. Some disclosures provide a use of the formulations and compositions disclosed herein for preventing or treating cancer.

### BRIEF DESCRIPTION OF FIGURES

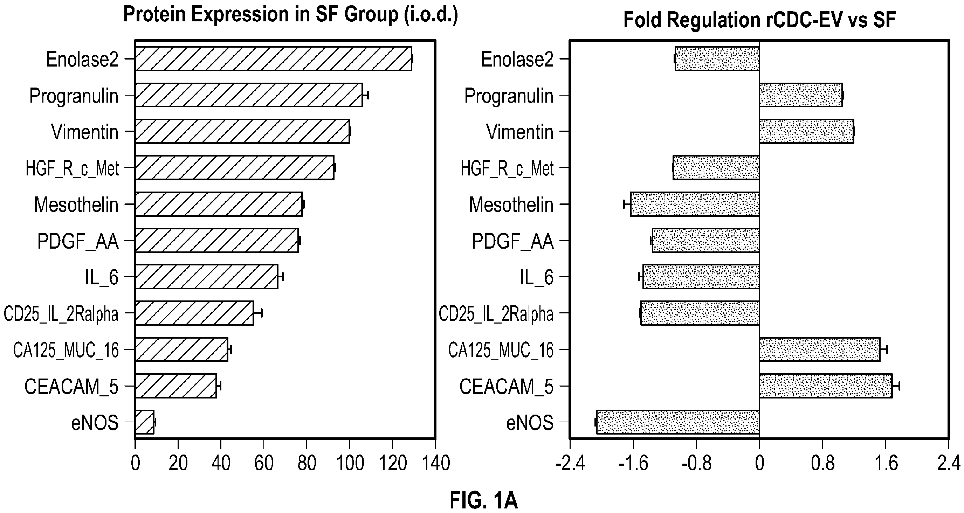

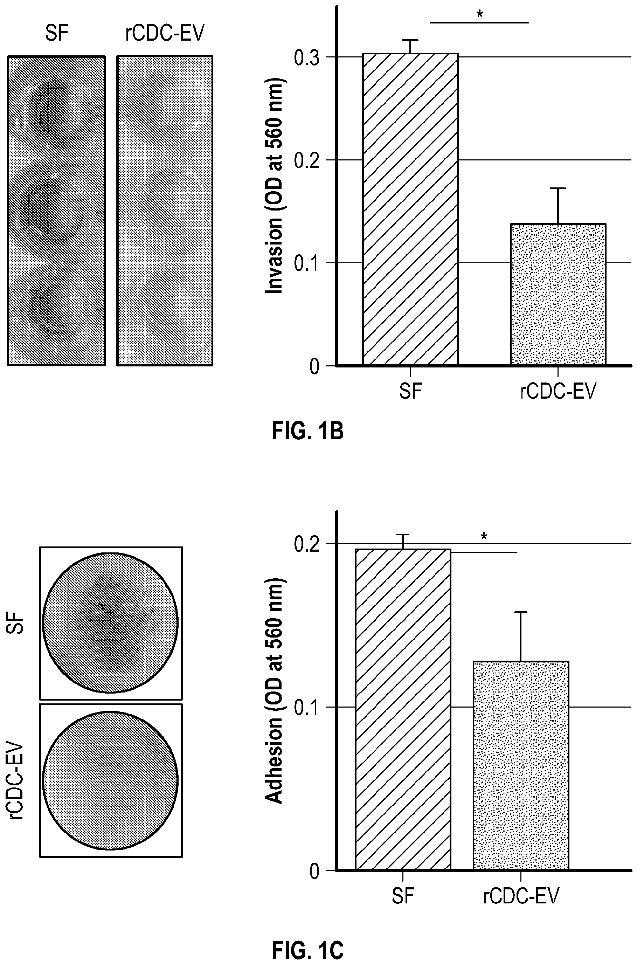

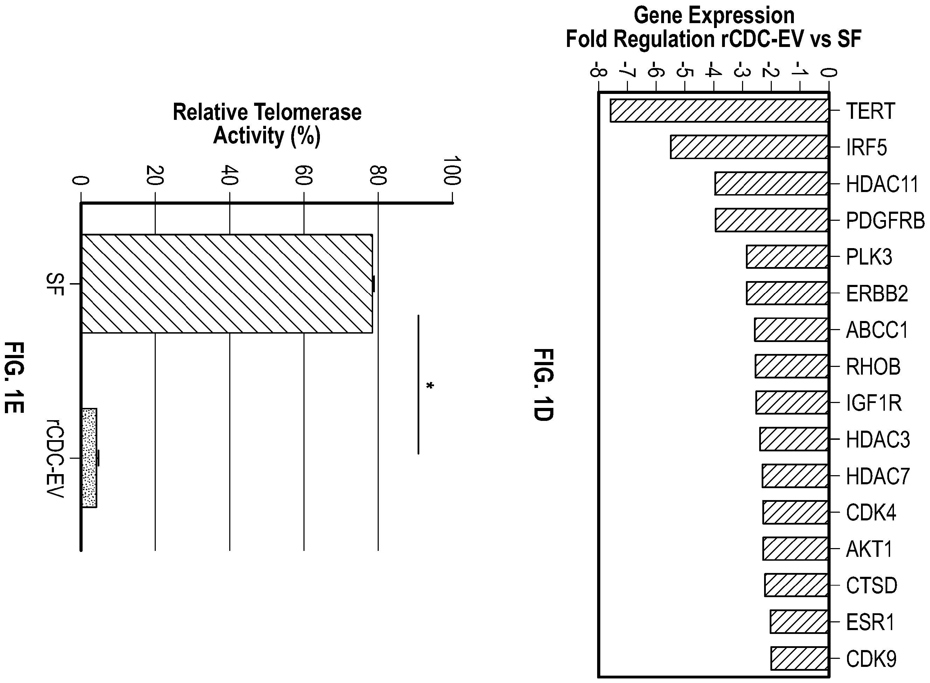

**Figure 1****. CDC-EVs negatively impact the aggressiveness of human HT1080 fibrosarcoma cells in vitro.** **Fig. 1A****.** Priming of HT1080 cells with CDC-EVs was associated with a favorably balanced modulation of cancer progression-related proteins compared to culture in serum-free media (SF) alone (grey bars). Only significantly modulated (p<0.05) proteins are shown. Black bars represent the protein expression levels in the SF cells. **Fig. 1B****.** Representative wells and a decreased invasion capacity of HT1080 after priming the cells with CDC-EVs vs SF. **Fig. 1C****.** Representative wells and a decreased adhesion capacity of HT1080 after priming the cells with CDC-EVs vs SF. **Fig. 1D****.** Priming of HT1080 cells with CDC-EVs was associated with a down-regulation of many "cancer drug target" genes. Only significant (p<0.05) and more than two-fold modulated genes are shown. **Fig. 1E****.** Telomerase activity in extracts of HT1080 cells was determined following telomeric repeat amplification protocol in SF and CDC-EV primed cells, showing a marked decrease in the later group of cells. *p<0.05. Bar graphs represent mean (±SEM). Minimum number of replicates per experiment was 3.

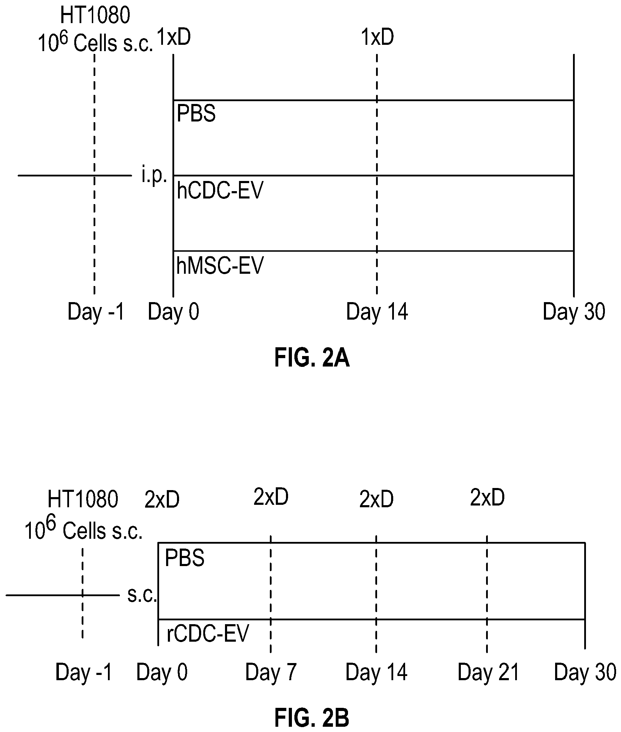

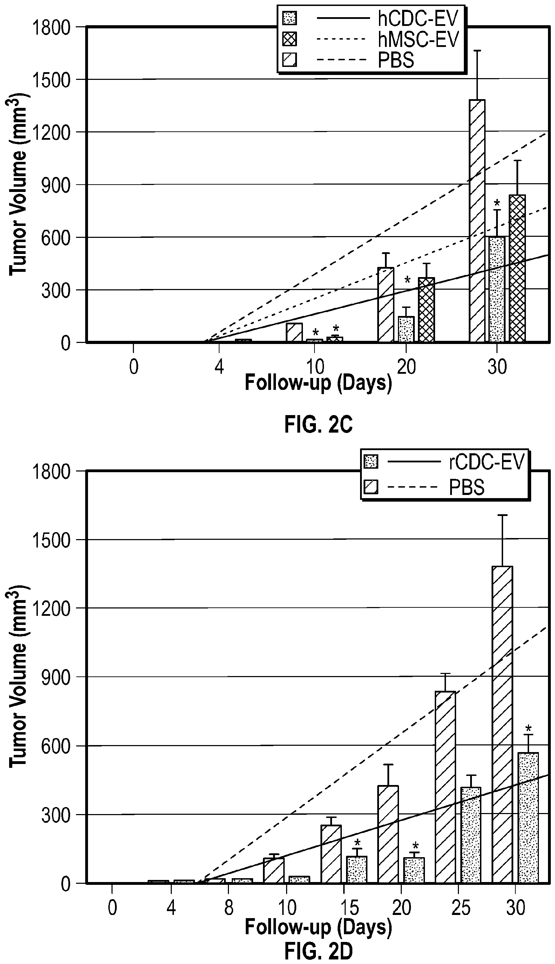

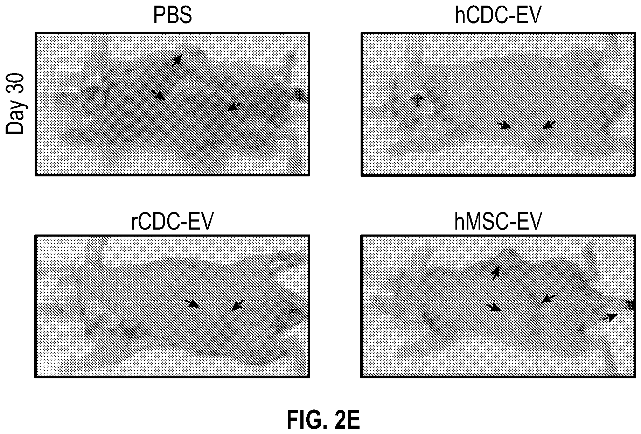

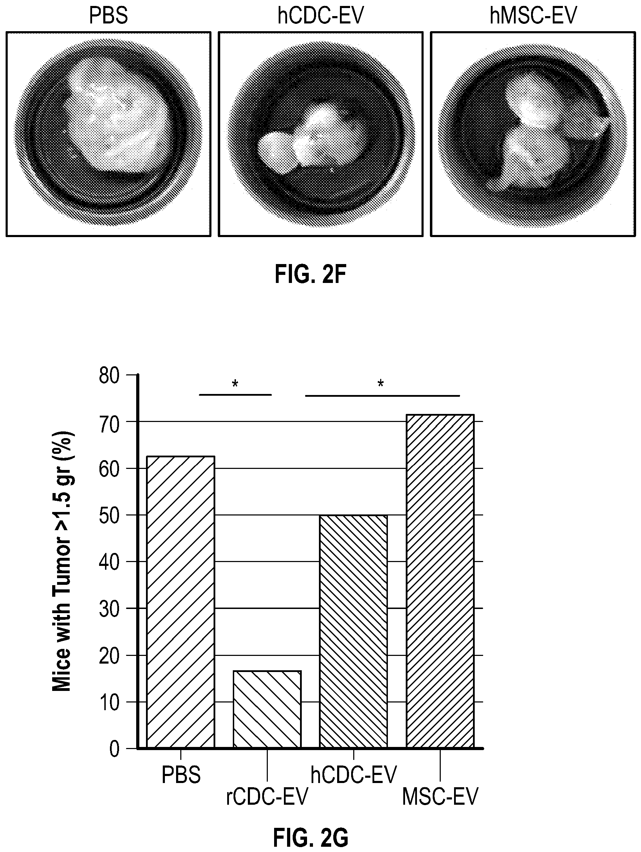

**Figure 2****. CDC-EVs decrease fibrosarcoma growth in mice.** **Fig. 2A****.** Study design where systemic (intraperitoneal -i.p.) delivery of human CDC-EVs (hCDC-EV or CDC-EV; n=6) was compared to PBS (n=9) and human MSC-EVs (hMSC-EV or MSC-EV; n=8) injections. 1xD refers to single dose. **Fig. 2B****.** Study design where local (subcutaneous, peritumoral - s.c.) delivery of rat CDC-EVs (rCDC-EV; n=6) was compared with local PBS (n=6) injections. 2xD refers to double dose. **Fig. 2C****.** External tumor growth measured with a caliper in the systemic-delivery protocol, showing a significant decrease in the hCDC-EV vs PBS groups. **Fig. 2D****.** External tumor growth measured with a caliper in the local-delivery protocol, showing a significant decrease in the rCDC-EV vs PBS groups. **Fig. 2E****.** Representative images of mice at day 30 with visibly smaller tumors (marked with arrows) in animals treated with human- and rat-CDC-EVs compared with the other two control groups (PBS and MSC-EV). **Fig. 2F****.** Representative images of the harvested tumors. **Fig. 2G****.** Bar graph showing the proportion of mice with the heaviest tumors (defined as tumor weigh more than the mean of 1. 5 gr in all mice together). * p<0.05. Tumor growth's bar graphs represent mean (±SEM).

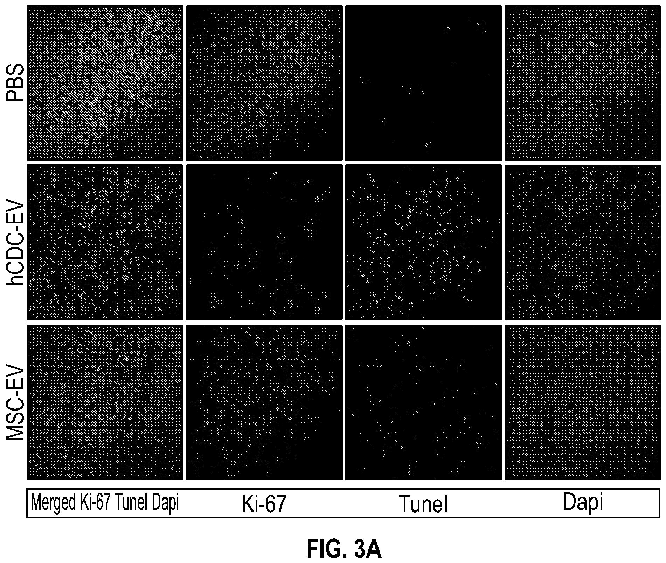

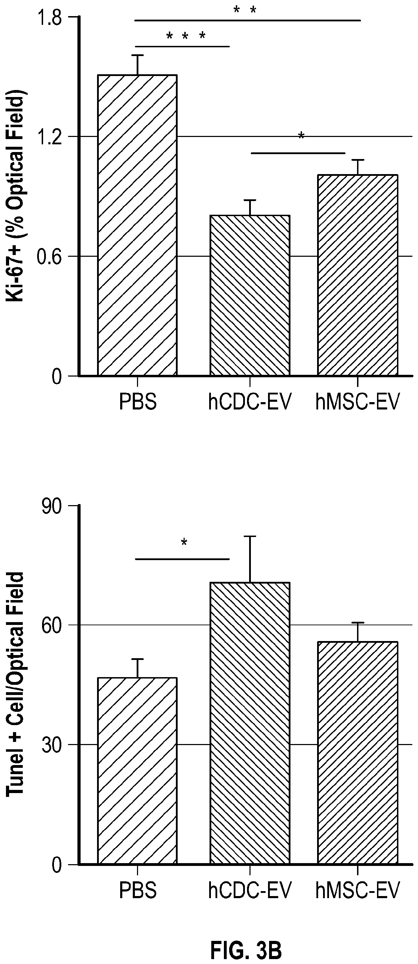

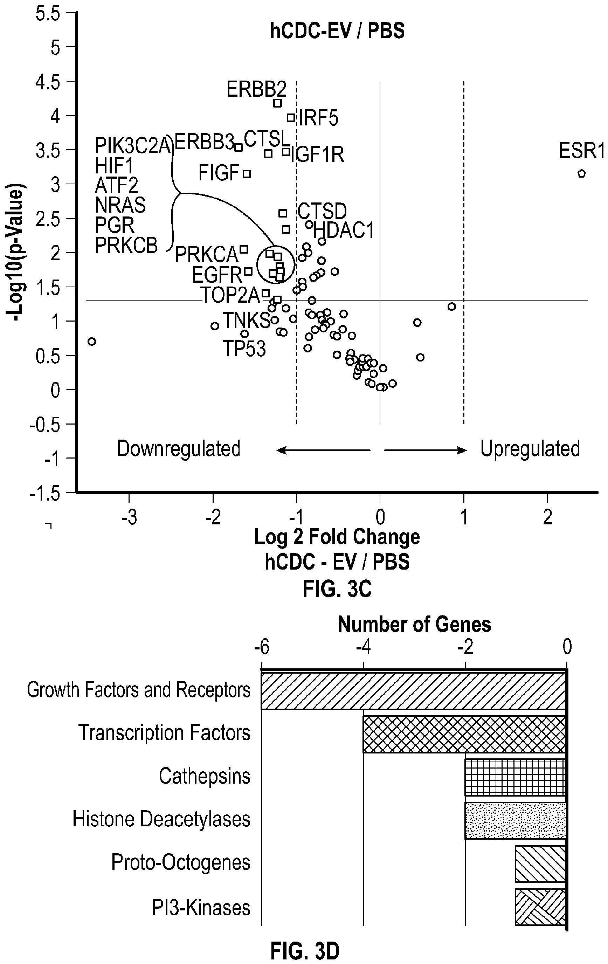

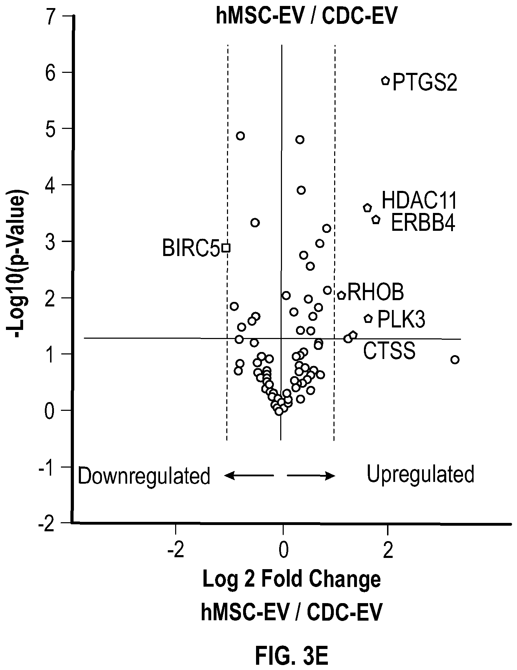

**Figure 3****. Local effects of the systemically delivered extracellular vesicles (EVs) at the tumor site in mice with fibrosarcoma.** **Fig. 3A****.** Immunostaining for Ki-67 and terminal deoxynucleotidyl transferase dUTP nick end labeling (TUNEL), showing a marked decrease of proliferation and increase of apoptosis in the CDC-EV treated (n=5) compared to PBS (n=5) injected mice. Differences in proliferation and apoptosis markers between CDC-EV and MSC-EV (n=6) treated mice were less marked. **Fig. 3B****.** Graphs showing differences in expression of Ki-67 (upper panel) and TUNEL (lower panel) between groups. Fig. 3C. Volcano plot representing gene expression in CDC-EV vs PBS-treated mice. Genes with significant and higher than two-fold down- (lighter grey) and up-regulation (darker grey) are referenced. **Fig. 3D****.** Schematic representation of the distribution of the highly (more than twofold) down-regulated genes in CDC-EV vs PBS groups. **Fig. 3E****.** Volcano plot representing gene expression in MSC-EV vs CDC-EV-treated mice. Genes with significant and higher than two-fold down-(green) and up-regulation (red) are referenced. * p<0.05, **p<0.01, ***p<0.001. Bar graphs represent mean (±SEM).

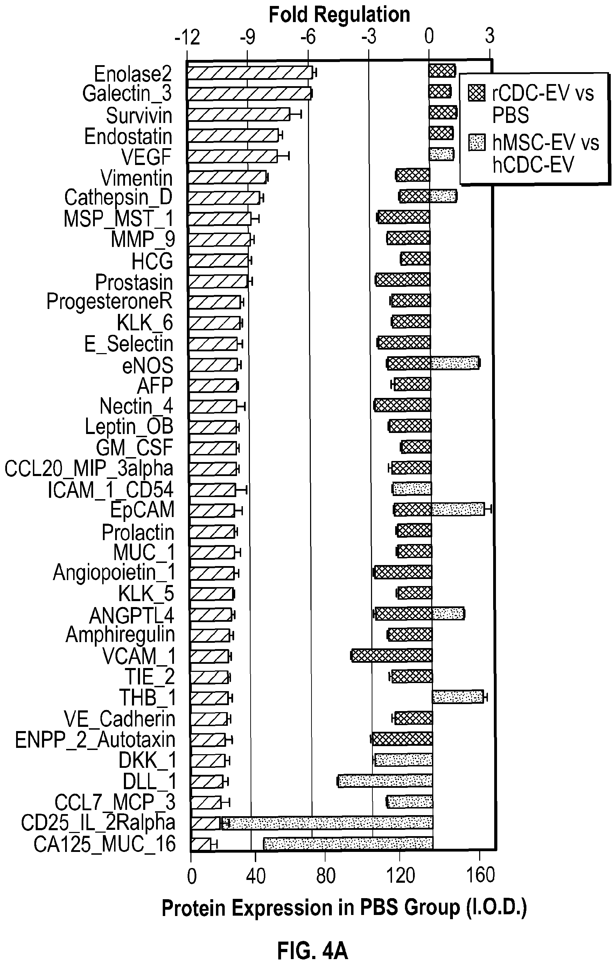

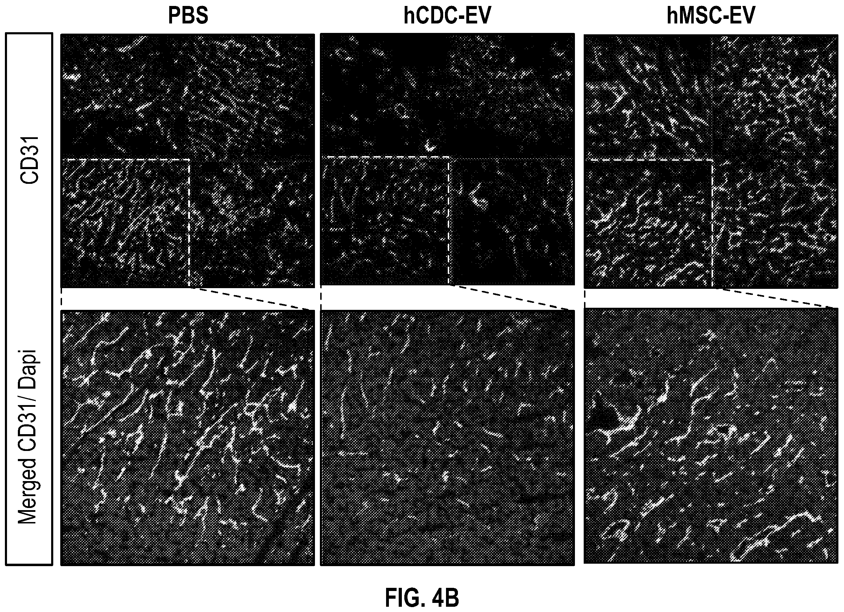

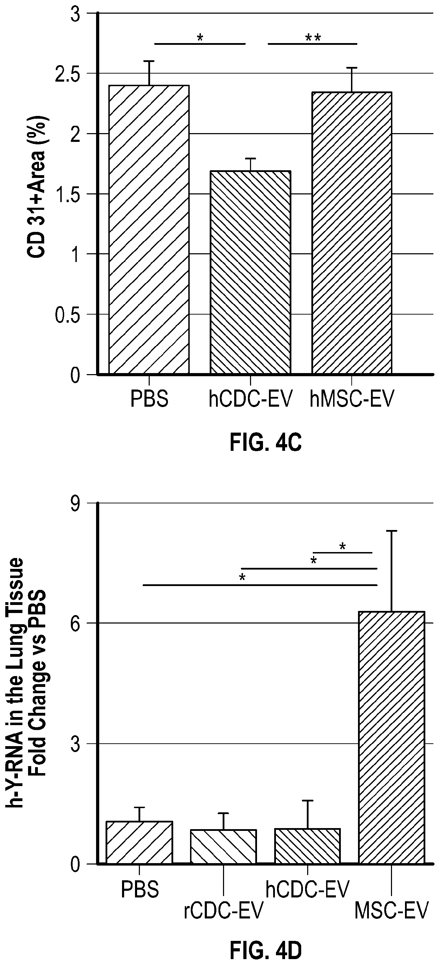

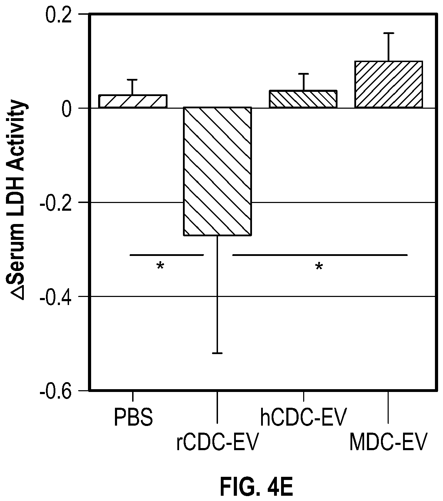

**Figure 4****. Tumor vascularization and lung metastases are attenuated by the treatment with CDC-EVs in mice with fibrosarcoma.** **Fig. 4A****.** Cancer-related proteins' expression at the local tumor in the PBS (n=6) injected mice (black bars). Colored bars show the foldregulation in proteins' level in the CDC-EV (n=6) vs PBS-injected (grey) and MSC-EV (n=4) vs human CDC-EV-treated mice (lighter grey and marked with an "x"). Only proteins with significant (p<0.05) differences between groups are shown. **Fig. 4B****.** Immunostaining for endothelial cell marker CD31 shows lower tumor vascularization in the CDC-EVs-treated mice (n=5) compared with other control groups (PBS, n=5 and MSC-EVs, n=6). Each grey-framed square in the upper row of images corresponds to one animal, thus sections from four animals per group are jointly shown in the upper pictures. **Fig. 4C****.** Graph showing differences in expression of CD-31 in CDC-EV treated mice and both control groups. **Fig. 4D****.** Presence of HT1080 fibrosarcoma cells in the whole mice lung lysates was analyzed by measurement of the expression of the human Y-RNA fragment normalized for the expression of mice U6 with q-PCR. The results reveal higher presence of cancer cells in the lungs of MSC-EV-treated mice (n=6) compared to the remaining groups (PBS, n=8; hCDC-EV, n=5; rCDC-EV, n=4). **Fig. 4E****.** Changes in serum LDH activity were measured at days 18 and 25, showing significant decrease in the rCDC-EV-treated mice (n=4) compared to PBS (n=8) and MSC-EV (n=5) groups. The number of mice in the hCDC-EV was 4. * p <0.05, ** p<0.01. Bar graphs represent mean (±SEM).

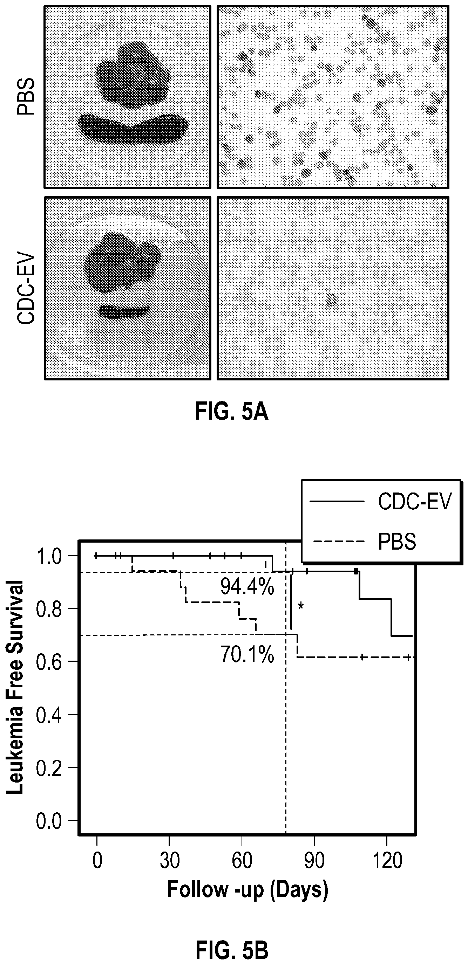

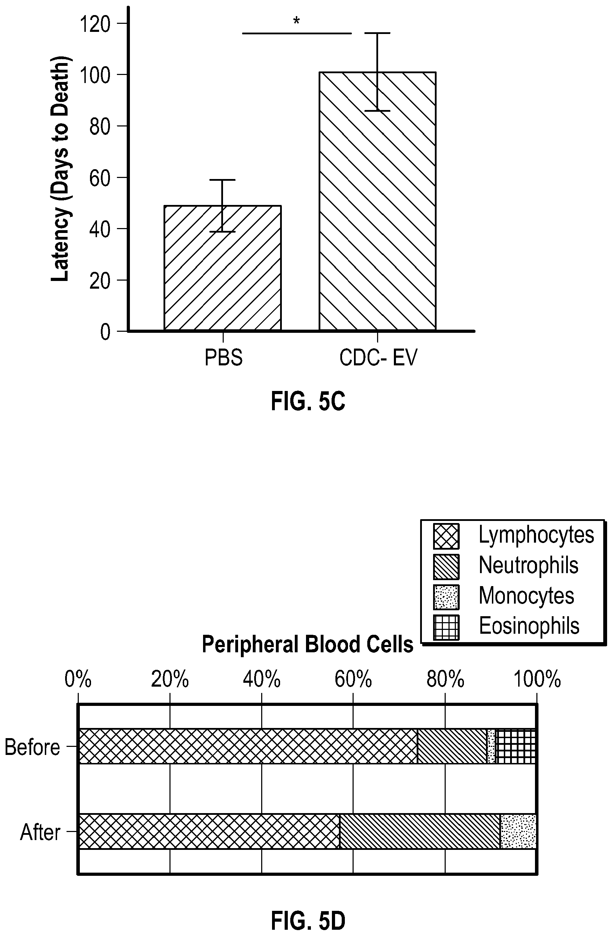

**Figure 5****. CDC-EVs decrease the incidence of spontaneous leukemia and increase the survival in old rats.** **Fig. 5A****.** Representative images showing marked splenomegaly and increased number of immature, enlarged lymphoid cells in the blood smear of a rat from PBS group in contrast with a normal spleen and blood smear in a CDC-EV-treated rat. **Fig. 5B****.** Kaplan-Meier leukemia-free survival curves in CDC-EV (n=24) and PBS (n=20) rats. **Fig. 5C****.** The latency to leukemia-related mortality was doubled in CDC-EV rats. **Fig. 5D****.** Changes in proportional distribution of white blood cells in peripheral blood in a leukemic CDC-EV rat one-week after administration of EVs. * p<0.05. Bar graph represent mean (±SD).

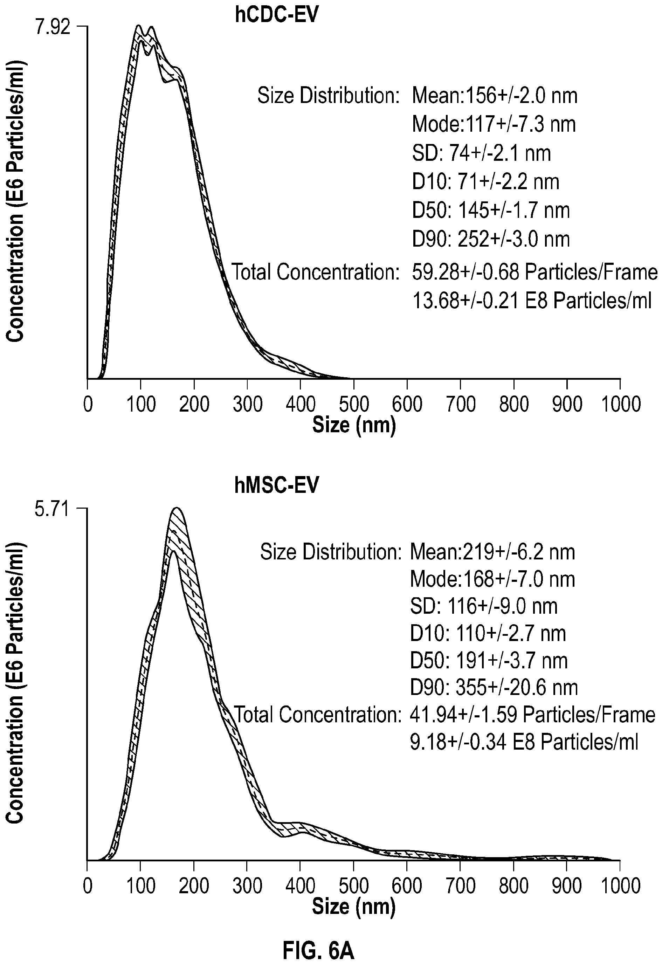

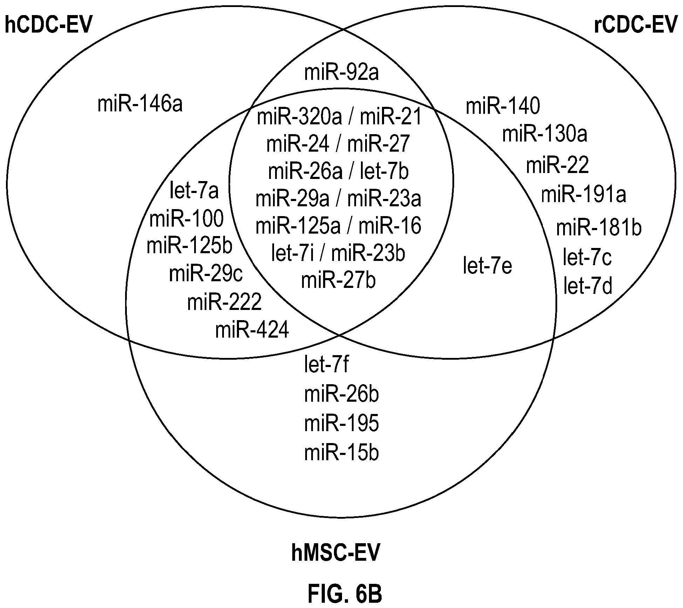

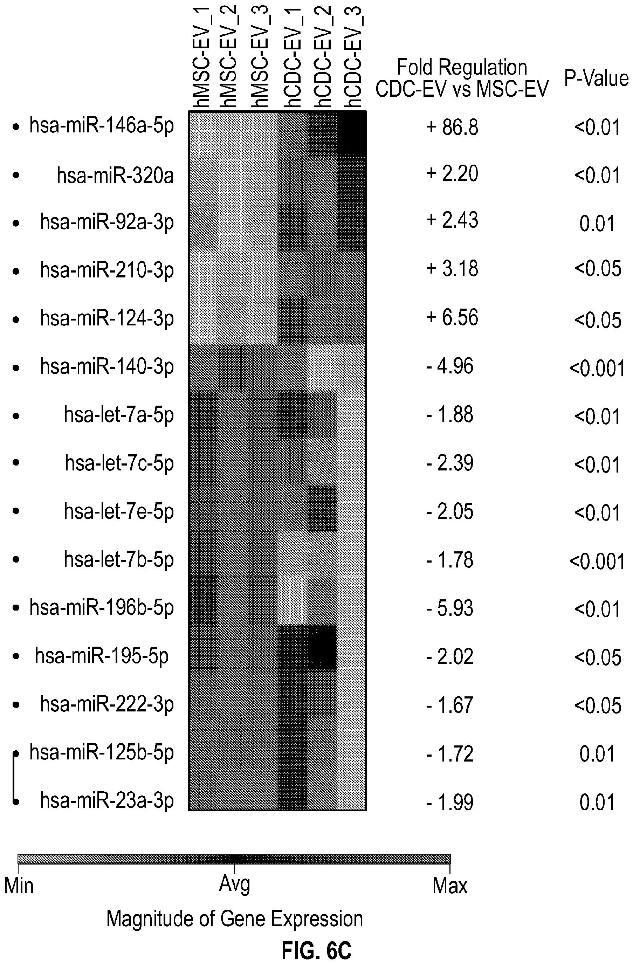

**Figure 6****. Differences between EVs secreted by the cardiosphere-derived cells (CDCs), and bone marrow-derived mesenchymial stem cells (MSCs).** **Fig. 6A****.** Size distribution and EVs number secreted by the CDCs (panel i) and MSCs (panel ii) measured by Nanoparticles Tracking Analysis. **Fig. 6B****.** Distribution of most abundant micro-RNA (miRs) in human- (hCDC-EV), rat-CDC-EV (rCDC-EV) and human MSC-EVs analyzed with an array. **Fig. 6C****.** Cluster map with differentially expressed miRs (abundant and non-abundant) in human CDC-EVs vs MSC-EVs.

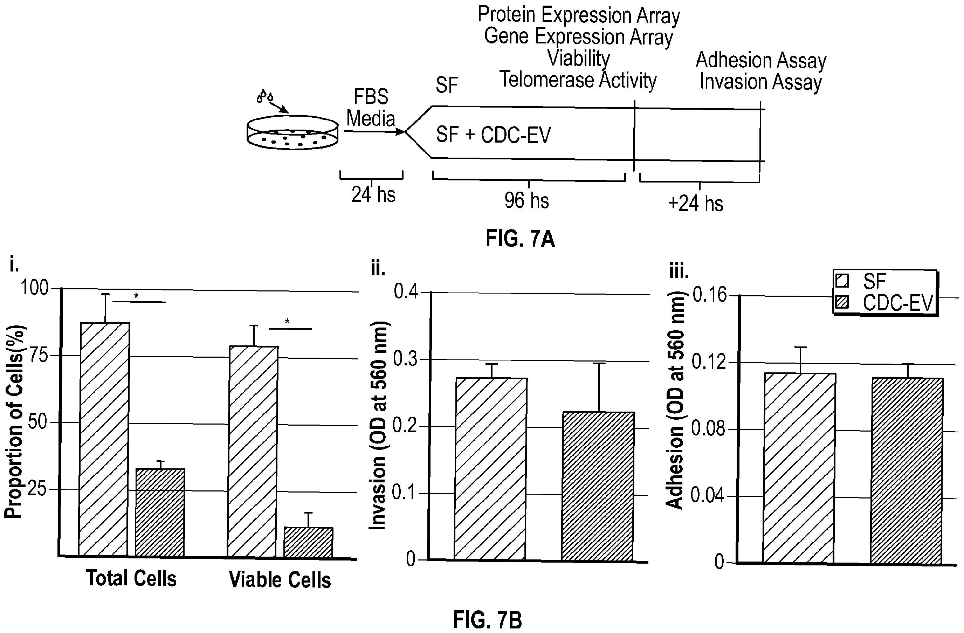

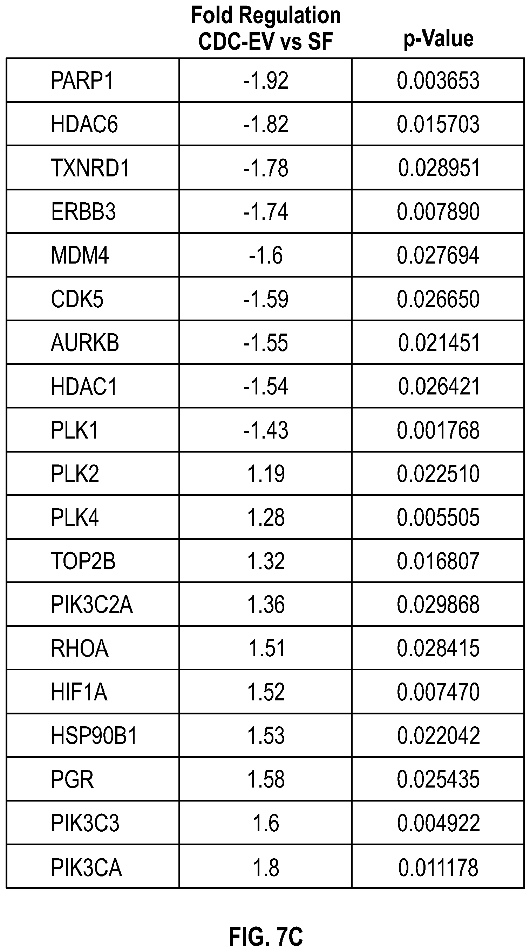

**Figure 7****.** **Fig. 7A****.** Protocol used for the in vitro studies. **Fig. 7B****.** NCl-H23 neuroblastoma cells were less viable after priming them with EVs secreted by the CDC-EVs vs SF (panel i). Invasion and adhesion were not significantly affected (panels ii and iii, respectively). **Fig. 7C****.** The complete list of significantly modulated genes after priming HT1080 fibrosarcoma cells with CDC-EVs compared to culture them in SF alone. *p<0.05. Bar graphs represent mean (±SEM).

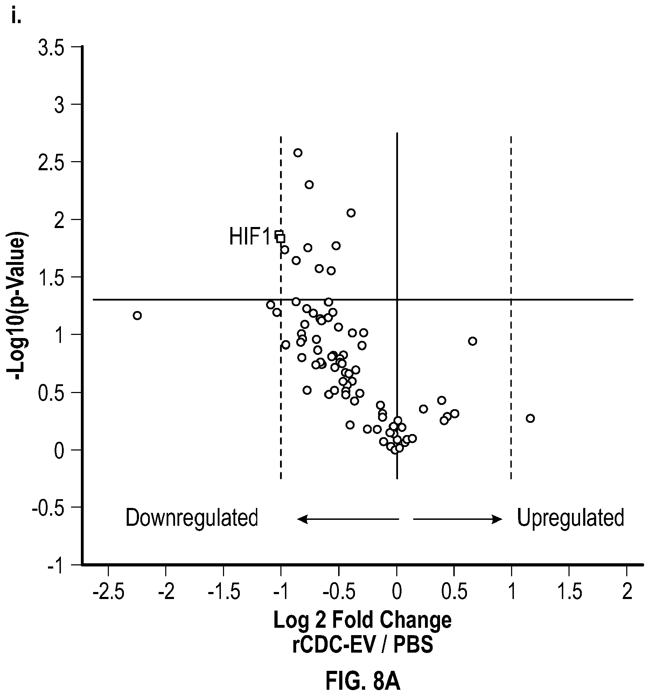

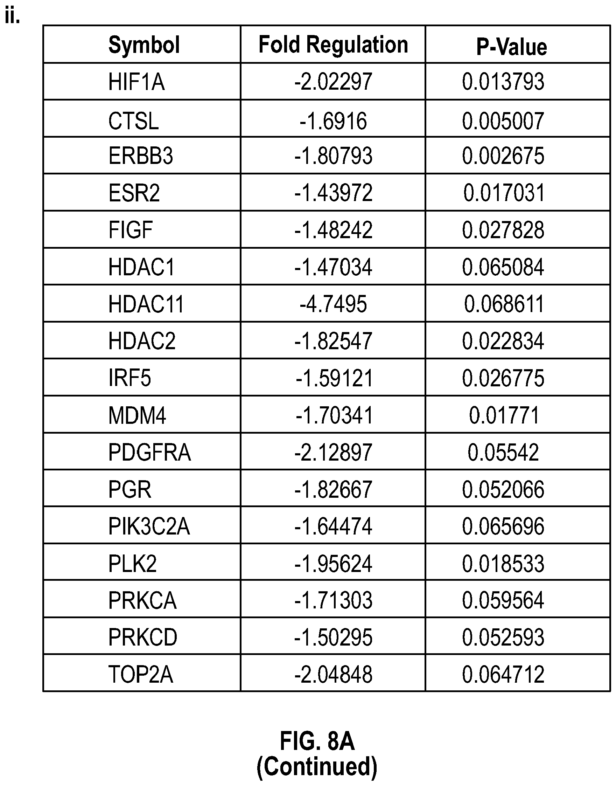

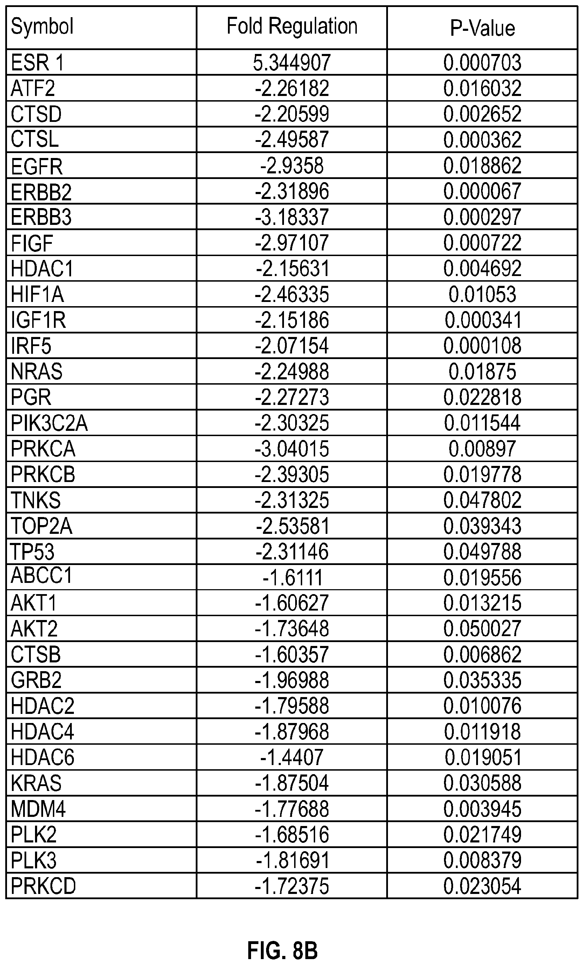

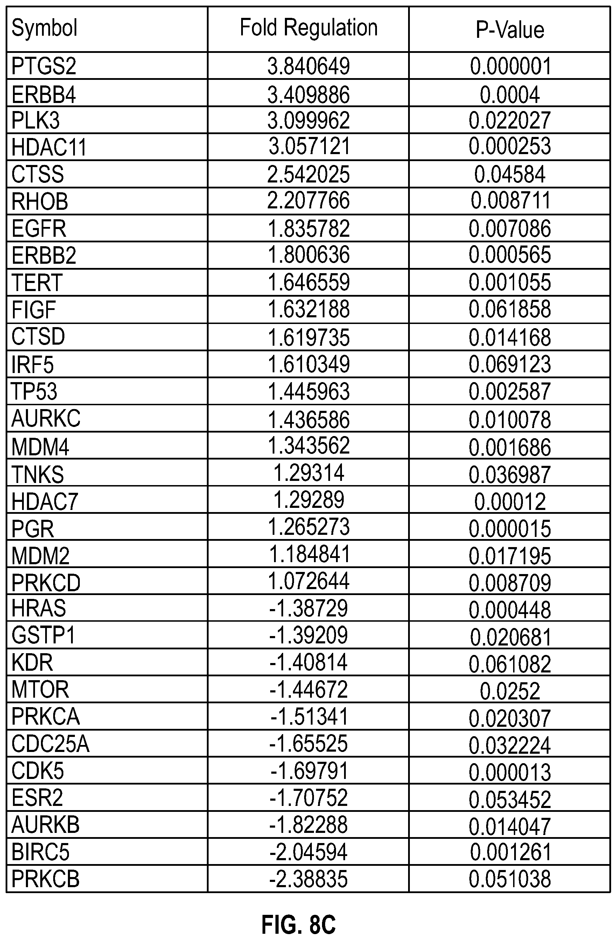

**Figure 8****.** Transcriptional changes at the tumor site in mice with fibrosarcoma. Fig. 8Ai. Volcano plot representing gene expression in rat CDC-EV vs PBS-treated mice. HIF1 was the only gene with a significant and higher than two-fold down-regulation. No up-regulated genes were detected. Fig. 8Aii. The complete list of significantly modulated genes in rat CDC-EV vs PBS groups. **Fig. 8B****.** The complete list of significantly modulated genes in human CDC-EV vs PBS groups. **Fig. 8C****.** The complete list of significantly modulated genes in human MSC-EV vs CDC-EV groups. Genes with borderline significance changes are included in the lists as well.

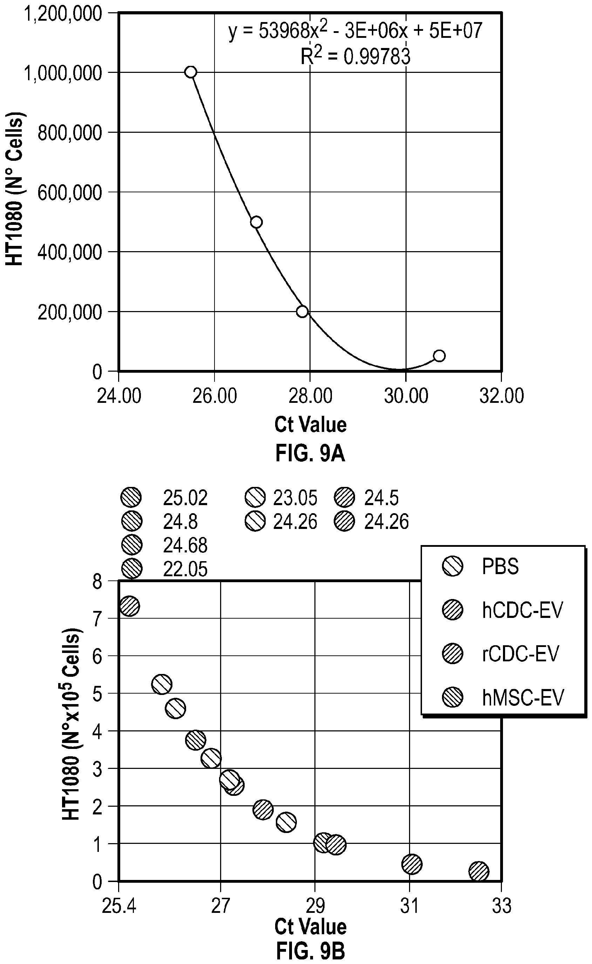

**Figure 9****.** Presence of HT1080 fibrosarcoma cells in lung tissue analyzed by measuring the expression of human Y-RNA fragment with q-PCR. **Fig. 9A****.** Standard curve was built using increasing numbers of pure HT1080 cells (range 10³ to 10⁶). **Fig. 9B****.** Plotted results correlating the number of HT1080 cells (Y-axis) with the Ct-values for human Y-RNA expression (X-axis) obtained in the lung tissue of different mice. In some cases, the results fell out of the lower Ct-limit of the standard curve, these are shown out of the plot and individual Ct values are referenced.

### DETAILED DESCRIPTION

Described herein are methods of treating a cancer and/or one or more disease states associated therewith. In some embodiments, the method of treating a cancer and/or one or more disease states associated therewith includes administering a therapeutically effective amount of one or more of cardiosphere-derived cells (CDCs), extracellular vesicles (EVs) derived from CDCs (CDC-EVs), exosomes derived from CDCs (CDC-XOs), and/or combinations thereof to a patient in need thereof. Some embodiments pertain to methods and compositions related to an anti-oncogenic effect of CDCs and extracellular vesicles (EVs) from heart-derived cells (e.g., one or more of CDCs or cardiospheres). In some embodiments, the EVs are CDC-EVs. In some embodiments, CDCs and CDC-EVs are used for the treatment of cancer.

CDCs are a population of cells generated by manipulating cardiospheres, cultured cells initially obtained from heart sample biopsies, subsequently cultured as explants and suspension cultured cardiospheres. For example, CDCs can be generated by plating cardiospheres on a solid surface which is coated with a substance which encourages adherence of cells to a solid surface of a culture vessel, *e.g*., fibronectin, a hydrogel, a polymer, laminin, serum, collagen, gelatin, or poly-D-lysine, and expanding same as an adherent monolayer culture. CDCs can be repeatedly passaged, *e.g.*, passaged two times or more, according to standard cell culturing methods.

EVs include lipid bilayer structures generated by cells, and include XOs (XOs), microvesicles (MVs), membrane particles, membrane vesicles, exosome-like vesicles, ectosomes, ectosome-like vesicles, or exovesicles. XOs are vesicles formed via a specific intracellular pathway involving multivesicular bodies or endosomal-related regions of the plasma membrane of a cell. Their lipid membrane is typically rich in cholesterol and contains sphingomyelin, ceramide, lipid rafts and exposed phosphatidylserine. XOs express certain marker proteins, such as integrins and cell adhesion molecules, but generally lack markers of lysosomes, mitochondria, or caveolae. In some embodiments, the XOs contain cell-derived components, such as but not limited to, proteins, DNA and RNA (*e.g*., microRNA and noncoding RNA). In some embodiments, EVs can be obtained from cells obtained from a source that is allogeneic, autologous, xenogeneic, or syngeneic with respect to the recipient of the XOs.

Certain types of RNA, *e.g*., microRNA (miRNA), which make up a portion of the molecule cargo of the vesicle, are known to be carried by XOs and EVs. miRNAs function as post-transcriptional regulators, often through binding to complementary sequences on target messenger RNA transcripts (mRNAs), thereby resulting in translational repression, target mRNA degradation and/or gene silencing. For example, miR146a exhibits over a 250-fold increased expression in CDCs, and miR210 is upregulated approximately 30-fold, as compared to the XOs isolated from normal human dermal fibroblasts. While in some embodiments, the therapeutic compositions can include CDCs, CDC-EVs, CDC-MVs, and/or CDC-XOs, in other embodiments, a therapeutic composition can lack CDCs and/or vesicles and instead includes a composition with an effective amount of RNA polynucleotide or vector encoding RNA polynucleotide or the molecular cargo of a vesical.

In some embodiments, the described EVs molecular contents are based on parental cellular origin and regulatory state at time of formation. In some embodiments, generic budding formation and release mechanisms establish a common set of features of the molecular content of EVs as a consequence of their origin, such as endosome-associated proteins (e.g., Rab GTPase, SNAREs, Annexins, and flotillin), proteins that are known to cluster into microdomains at the plasma membrane or at endosomes (four transmembrane domain tetraspanins, e.g., CD63, CD81, CD82, CD53, and CD37), lipid raft associated proteins (e.g., glycosylphosphatidylinositol- anchored proteins and flotillin), cholesterol, sphingomyelin, and hexosylceramides.

As used herein, "and/or" refers to and encompasses any and all possible combinations of one or more of the associated listed items, as well as the lack of combinations when interpreted in the alternative ("or").

"Treat" or "treating" or "treatment" refers to any type of action that imparts a modulating effect, which, for example, can be a beneficial effect, to a subject afflicted with a disorder, disease or illness, including preventing the manifestation of disease states associated with the condition, improvement in the condition of the subject (e.g., in one or more symptoms or in the disease), delay or reduction in the progression of the condition, and/or change in clinical parameters, disease or illness, curing the illness, etc.

The term "therapeutically effective amount," as used herein, refers to an amount of the therapeutic (e.g., CDC-XOs, CDC-MVs, CDC-EVs, CDCs, XOs, MVs, EVs, molecular cargo of EVs (including XOs or MVs), and/or combinations thereof) that imparts a modulating effect, which, for example, can be a beneficial effect, to a subject afflicted with a disorder, disease or illness, including improvement in the condition of the subject (e.g., modulating one or more symptoms), delay or reduction in the progression of the condition, prevention or delay of the onset of the disorder, and/or change in clinical parameters, disease or illness, etc. For example, in some embodiments, an effective amount can refer to the amount of a composition, compound, or agent that improves a condition in a subject by at least 5%, e.g., at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100%. Actual dosage levels of active ingredients and agents in an active composition of the disclosed subject matter can be varied so as to administer an amount of the active agent(s) that is effective to achieve the desired response for a particular subject and/or application. The selected dosage level will depend upon a variety of factors including, but not limited to, the activity of the composition, formulation, route of administration, combination with other drugs or treatments, severity of the condition being treated, and the physical condition and prior medical history of the subject being treated. Determination and adjustment of an effective dose, as well as evaluation of when and how to make such adjustments, are contemplated herein. The term "a therapeutically effective amount" can mean an amount of CDC-XOs, CDC-EVs, CDCs, EVs, and/or molecular cargo EVs (including XOs and/or MVs) sufficient to prevent the spread of or reverse cancer.

The "patient" or "subject" treated as disclosed herein is, in some embodiments, a human patient, although it is to be understood that the principles of the presently disclosed subject matter indicate that the presently disclosed subject matter is effective with respect to all vertebrate species, including mammals, which are intended to be included in the terms "subject" and "patient." Suitable subjects are generally mammalian subjects. The subject matter described herein finds use in research as well as veterinary and medical applications. The term "mammal" as used herein includes, but is not limited to, humans, non-human primates, cattle, sheep, goats, pigs, horses, cats, dog, rabbits, rodents (e.g., rats or mice), monkeys, etc. Human subjects include neonates, infants, juveniles, adults and geriatric subjects.

Cardiospheres are undifferentiated cardiac cells that grow as self-adherent clusters. Briefly, heart tissue can be collected from a patient during surgery or cardiac biopsy. In some embodiments, heart tissue can be harvested from the left ventricle, right ventricle, septum, left atrium, right atrium, crista terminalis, right ventricular endocardium, septal or ventricle wall, atrial appendages, or combinations thereof. In some embodiments, a biopsy can be obtained, for example, using a percutaneous bioptome as described in US/2009/012422 and US/2012/0039857.

In some embodiments, the tissue can cultured directly, or alternatively, the heart tissue can be frozen, thawed, and then cultured. In some embodiments, the tissue can be digested with protease enzymes such as collagenase, trypsin and the like. In some embodiments, the heart tissue can be cultured as an explant such that cells including fibroblast-like cells and cardiosphere-forming cells grow out from the explant. In some embodiments, an explant is cultured on a culture vessel coated with one or more components of the extracellular matrix (e.g., fibronectin, laminin, collagen, elastin, or other extracellular matrix proteins). In some embodiments, the tissue explant can be cultured for about 1, 2, 3, 4, or more weeks prior to collecting the cardiosphere-forming cells. In some embodiments, a layer of fibroblast-like cells can grow from the explant onto which cardiosphere-forming cells appear. In some embodiments, cardiosphere-forming cells can appear as small, round, phase-bright cells under phase contrast microscopy. In some embodiments, cells surrounding the explant including cardiosphere-forming cells can be collected by manual methods or by enzymatic digestion. In some embodiments, the collected cardiosphere-forming cells can be cultured under conditions to promote the formation of cardiospheres. In some embodiments, the cells are cultured in cardiosphere-growth medium comprising buffered media, amino acids, nutrients, serum or serum replacement, growth factors including but not limited to EGF and bFGF, cytokines including but not limited to cardiotrophin, and other cardiosphere promoting factors such as but not limited to thrombin. In some embodiments, cardiosphere-forming cells can be plated at an appropriate density necessary for cardiosphere formation, such as about 20,000-100,000 cells/mL. In some embodiments, the cells can be cultured on sterile dishes coated with poly-D-lysine, or other natural or synthetic molecules that hinder the cells from attaching to the surface of the dish. In some embodiments, cardiospheres can appear spontaneously about 2-7 days or more after cardiosphere-forming cells are plated.

CDCs are a population of cells generated by manipulating cardiospheres. In some embodiments, CDCs can be generated by plating cardiospheres on a solid surface which is coated with a substance which encourages adherence of cells to a solid surface of a culture vessel (e.g., fibronectin, a hydrogel, a polymer, laminin, serum, collagen, gelatin, or poly-D-lysine) and expanding same as an adherent monolayer culture. In some embodiments, CDCs can be repeatedly passaged (e.g., passaged two times or more) according to standard cell culturing methods.

XOs are vesicles formed via a specific intracellular pathway involving multivesicular bodies or endosomal-related regions of the plasma membrane of a cell. XOs can range in size from approximately 20-150 nm in diameter. In some embodiments, XOs have a characteristic buoyant density of approximately 1.1-1.2 g/mL, and a characteristic lipid composition. In some embodiments, XOs can be isolated based on their size and buoyancy. XOs lipid membrane is typically rich in cholesterol and contains sphingomyelin, ceramide, lipid rafts and exposed phosphatidylserine. In some embodiments, XOs express certain marker proteins, such as integrins and cell adhesion molecules. In some embodiments, XOs generally lack markers of lysosomes, mitochondria, or caveolae. In some embodiments, XOs contain cell-derived components, such as but not limited to, proteins, DNA and RNA (e.g., microRNA and noncoding RNA). In some embodiments, XOs are isolated from other EVs and/or from cellular debris using one or more features of the XOs. In some embodiments, XOs can be obtained from cells obtained from a source that is allogeneic, autologous, xenogeneic, or syngeneic with respect to the recipient of the XOs.

Certain types of RNA, e.g., microRNA (miRNA), are enriched or are carried by XOs. In some embodiments, miRNAs function as post-transcriptional regulators, often through binding to complementary sequences on target messenger RNA transcripts (mRNAs), thereby resulting in translational repression, target mRNA degradation and/or gene silencing. In some embodiments, miR146a exhibits over a 250-fold increased expression in XOs from CDCs, and miR210 is upregulated approximately 30-fold in XOs from CDCs, as compared to the XOs isolated from normal human dermal fibroblasts.

Methods for preparing XOs can include (or lack) one or more of the following steps: culturing cardiospheres or CDCs in conditioned media, isolating the cells from the conditioned media, purifying the exosome (e.g., by sequential centrifugation, etc.), optionally, clarifying the XOs on a density gradient (e.g., a sucrose density gradient). In some embodiments, the isolated and purified XOs are essentially free of non-exosome components, such as components of cardiospheres or CDCs. In some embodiments, XOs can be resuspended in a buffer such as a sterile PBS buffer containing 0.01-1% human serum albumin. In some embodiments, the XOs may be frozen and stored for future use.

In some embodiments, XOs can be prepared using a commercial kit such as, but not limited to the ExoSpin^{™} Exosome Purification Kit, Invitrogen^{®} Total Exosome Purification Kit, PureExo^{®} Exosome Isolation Kit, and ExoCap^{™} Exosome Isolation kit. In some embodiments, methods for isolating XOs from stem cells are found in US/2012/0093885 and US/2014/0004601. In some embodiments, collected XOs can be concentrated and/or purified using methods as disclosed elsewhere herein. In some embodiments, specific methodologies include, but are not limited to, ultracentrifugation, density gradient, HPLC, adherence to substrate based on affinity, or filtration based on size exclusion.

In some embodiments, for example, differential ultracentrifugation has become a leading technique wherein secreted XOs are isolated from the supernatants of cultured cells. In some embodiments, this approach allows for separation of XOs from nonmembranous particles, by exploiting their relatively low buoyant density. In some embodiments, size exclusion allows for their separation from biochemically similar, but biophysically different MVs, which possess larger diameters of (e.g., up to 1,000 nm). In some embodiments, differences in flotation velocity further allows for separation of differentially sized XOs. In some embodiments, XOs sizes will possess a diameter ranging from 30-200 nm, including sizes of 40-100 nm. In some embodiments, purification may rely on specific properties of the particular XOs of interest. In some embodiments, this includes, e.g., use of immunoadsorption with a protein of interest to select specific vesicles with exoplasmic or outward orientations. In some embodiments, more than one purification technique can be used together.

In some embodiments, one or more of differential centrifugation, discontinuous density gradients, immunoaffinity, ultrafiltration and high performance liquid chromatography (HPLC), differential ultracentrifugation can be used for exosome isolation. In some embodiments, the centrifugation technique utilizes increasing centrifugal force from 2000xg to 10,000xg to separate the medium- and larger-sized particles and cell debris from the exosome pellet at 100,000xg. In some embodiments, centrifugation alone allows for significant separation/collection of XOs from a conditioned medium. In some embodiments, enhanced specificity of exosome purification may deploy sequential centrifugation in combination with ultrafiltration, or equilibrium density gradient centrifugation in a sucrose density gradient, to provide for the greater purity of the exosome preparation (flotation density 1.1-1.2g/mL) or application of a discrete sugar cushion in preparation.

In some embodiments, ultrafiltration can be used to purify XOs without compromising their biological activity. In some embodiments, membranes with different pore sizes - such as 100 kDa molecular weight cut-off (MWCO) and gel filtration to eliminate smaller particles - have been used to avoid the use of a nonneutral pH or non-physiological salt concentration. In some embodiments, tangential flow filtration (TFF) systems are scalable (to >10,000L), allowing one to not only purify, but concentrate the exosome fractions, and such approaches are less time consuming than differential centrifugation. HPLC can also be used to purify XOs to homogeneouslysized particles and preserve their biological activity as the preparation is maintained at a physiological pH and salt concentration.

In some embodiments, other chemical methods exploit differential solubility of XOs for precipitation techniques, addition to volume-excluding polymers (*e.g*., polyethylene glycols (PEGs)), possibly combined additional rounds of centrifugation or filtration. In some embodiments, for example, a precipitation reagent, ExoQuick^{®}, can be added to conditioned cell media to quickly and rapidly precipitate a population of XOs. In some embodiments, flow field-flow fractionation (F1FFF) is an elution-based technique that is used to separate and characterize macromolecules (*e.g*., proteins) and nano- to micro-sized particles (*e.g*., organelles and cells) and which can be successfully applied to fractionate XOs from culture media.

In some embodiments, focused techniques may be applied to isolate specific XOs of interest. In some embodiments, this includes relying on antibody immunoaffinity to recognizing certain exosome-associated antigens. In some embodiments, XOs further express the extracellular domain of membrane-bound receptors at the surface of the membrane. In some embodiments, this presents a ripe opportunity for isolating and segregating XOs in connections with their parental cellular origin, based on a shared antigenic profile. In some embodiments, conjugation to magnetic beads, chromatography matrices, plates or microfluidic devices allows isolating of specific exosome populations of interest as may be related to their production from a parent cell of interest or associated cellular regulatory state. In some embodiments, other affinity-capture methods use lectins which bind to specific saccharide residues on the exosome surface.

The heart is known for its immunity to cancer. In contrast to other organs, the overall incidence of primary heart tumors was only 0.02% in an autopsy series in the United States, and only one-quarter of them were malignant. Terminal differentiation and low turnover of cardiomyocytes were proposed as the main mechanisms of heart resistance to tumor formation. However, this premise is brought into question by the facts that the heart contains a predominantly non-cardiomyocyte, proliferating population of fibroblasts, endothelial and vascular smooth muscle cells, which constitute ~70% of all cells in the adult heart. Moreover, the incidence of malignancies in the central nervous system, also characterized by a low rate of cell division, is much higher than that in heart. The unceasing and efficient oxygen-consuming metabolism of the heart, recently proposed as a potential anti-cancer mechanism, is challenged by the low incidence of cancer in many cardiac diseases related to altered oxygen supply-consumption balance (i.e. cyanotic congenital heart disease).

The concept of a tumor microenvironment has emerged as an important factor during primary tumor formation, as well as in later stages of invasion and metastasis. In some embodiments, disclosed herein are strategies that exploit the anti-cancer properties of the heart and microenvironment to achieve anti-cancer properties. In some embodiments, CDCs, CDC-EVs, CDC-MVs, CDC-XOs are used in methods of treating cancer. CDCs have demonstrated efficacy in various cardiac pathologies and no safety-related issues to date. The benefits of CDCs are mostly paracrine, mediated by nanoscale EVs, including XOs, which may themselves used as cell-free therapeutic candidates. WO/2014/028493 describes XOs derived from cardiosphere-derived cells (CDCs) and their therapeutic utility for the repair or regeneration of damaged or diseased cells or tissue, *e.g*., damaged cardiac tissue. US/2012/0315252 describes CDCs, their derivation from cardiospheres, and their therapeutic utility for increasing the function of a damaged or diseased heart of a mammal. WO/2005/012510 describes cardiospheres, their derivation from human or animal cardiac tissue biopsy samples, and their therapeutic utility in cell transplantation and functional repair of the myocardium or other organs.

Described herein are methods and compositions related to an anti-oncogenic effect of CDCs and CDC-derived EVs (e.g., CDC-EVs) or cardiosphere-derived EVs. For brevity, several embodiments are disclosed with reference to CDC and/or CDCs-EVs. It should be understood, however, that one or more of the treatments disclosed herein can be achieved with CDC-MVs, CDC-XOs, the isolated molecular cargo of CDCs-MVs, CDC-XOs, or CDC-EVs, and/or combinations thereof. Thus, in some embodiments, the methods of treatment described herein can be performed using one or more of CDCs, CDC-EVs, CDC-MVs, CDC-XOs, the isolated and/or purified molecular cargo of CDC-EVs, isolated and/or purified molecular cargo of CDC-MVs, isolated and/or purified molecular cargo of CDC-XOs, and/or combinations thereof. For example, in some embodiments, CDC-MVs are included in therapeutic mixtures with CDC-XOs (where EVs comprise XOs and MVs) and/or MVs are not removed from XOs. In other embodiments, XOs can be isolated from MVs so that the XOs are enriched and/or substantially free of MVs. In some embodiments, EVs, MVs, and XOs not derived from CDCs may be used in methods of treating cancer.

In some embodiments, differences in flotation velocity further allows for separation of differentially sized EVs. In some embodiments, the disclosed XOs have sizes (e.g., a diameter in nm) of less than or equal to about: 200, 150, 100, 40, 30, 10, or ranges including and/or spanning the aforementioned values. In some embodiments, XOs will possess a diameter ranging from 30-200 nm, including sizes of 40-100 nm. In some embodiments, the disclosed MVs and EVs have sizes (e.g., a diameter in nm) of greater than or equal to about: 1000, 750, 500, 400, 300, 250, 200, or ranges including and/or spanning the aforementioned values. In some embodiments, one or more methods disclosed herein involve the use of EVs having sizes (e.g., a diameter in nm) of greater than or equal to about: 1500, 1000, 750, 500, or ranges including and/or spanning the aforementioned values. In some embodiments, XOs have a characteristic buoyant density of approximately 1.1-1.2 g/mL, and a characteristic lipid composition.

In some embodiments, EV, MV, and/or XO isolation can be accomplished using biochemical and biophysical features for separation and analysis. In some embodiments, differential ultracentrifugation can be used as a technique wherein secreted EV, MV, and/or XO are isolated from the supernatants of cultured cells. In some embodiments, this approach allows for separation of EV, MV, and/or XO from nonmembranous particles, by exploiting their relatively low buoyant density. In some embodiments, differences in flotation velocity further allows for separation of differentially sized XOs. In some embodiments, size exclusion allows for their separation from biochemically similar, but biophysically different MVs, which possess larger diameters of up to 1,000 nm. As disclosed elsewhere herein, in some embodiments, MVs are also included in therapeutic mixtures with XOs (where EVs encompass both XOs and MVs) and/or MVs are not removed from XOs. Thus, separation techniques can be employed to provide a mixture of XOs and MVs. In other embodiments, XOs can be isolated from MVs so that the XOs are enriched and/or substantially free of MVs.

In some embodiments, methods for preparing XOs (or other EVs) can include the steps of: culturing cardiospheres or CDCs in conditioned media, isolating the cells from the conditioned media, purifying the exosome by, *e.g.*, sequential centrifugation, and optionally, clarifying the XOs (or other EVs) on a density gradient, *e.g*., sucrose density gradient. In some instances, the isolated and purified XOs are essentially free of non-exosome components, such as components of cardiospheres or CDCs. In some embodiments, XOs (or other EVs) can be re-suspended in a buffer such as a sterile PBS buffer containing 0.01-1% human serum albumin. The XOs (or other EVs) may be frozen and stored for future use.

In some embodiments, described herein is a method including isolating a biopsy specimen from a subject, culturing the biopsy specimen as an explant, generating explant derived cells (EDCs), culturing the EDCs into cardiospheres, and inducing formation of CDCs. In other embodiments, the method includes administering CDCs to a subject. In other embodiments, the method includes isolating XOs from the CDCs and administering CDC-derived EVs (including XOs) to a subject. In various embodiments, culturing the biopsy specimen as an explant includes mincing the biopsy specimen and culturing on a fibronectin coated vessel. In various embodiments, generating EDCs includes isolating cells from the explant. In various embodiments, isolated cells from the explant include loosely adherent cells and/or stromal-like cells. In various embodiments, culturing the EDCs into cardiospheres includes culturing of EDCs on poly-D-lysine dishes. In various embodiments, formation of CDCs includes culturing detached cardiospheres on a fibronectin coated vessel. Further examples and embodiments for CDC generation are described in U.S. Pat. Pub. No. 2008/0267921. In various embodiments, isolating CDC-derived XOs includes use of any of the techniques described herein. In various embodiments, administering CDCs to a subject includes use of any of the techniques described herein. In various embodiments, administering CDCs or CDC-derived XOs to a subject includes use of any of the techniques described herein. In various embodiments, the biopsy specimen is isolated from the same subject that is administered the CDCs or CDC-derived XOs. In various embodiments, biopsy specimen is isolated from a different subject that the subject that is administered the CDC-derived XOs.

In some embodiments, as disclosed elsewhere herein EVs can be used. In some embodiments, EVs originating from newt A1 cell line (Newt-EVs) are obtained after filtering A1 cell line CM containing EVs through a 10 KDa pore size filter following a similar process as for CDC-EV production. Newt-EVs are a non-cellular, filter sterilized product obtained from newt A1 cells cultured under defined, serum-free conditions. The final product, composed of secreted EVs and concentrated CM, is formulated in PlasmaLyte A and stored frozen. The frozen final product is ready to use for direct use (e.g., injection) after thawing.

In some embodiments, XOs can be prepared using a commercial kit such as, but not limited to the ExoSpin^{™} Exosome Purification Kit, Invitrogen^{®} Total Exosome Purification Kit, PureExo^{®} Exosome Isolation Kit, and ExoCap^{™} Exosome Isolation kit. In some embodiments, collected XOs can be concentrated and/or purified using, for example, ultracentrifugation, density gradients, HPLC, adherence to substrate based on affinity, or filtration based on size exclusion.

In some embodiments, differential ultracentrifugation can be used to isolate secreted XOs (and/or EVs) from the supernatants of cultured cells. In some embodiments, this approach allows for separation of XOs (and/or of EVs) from nonmembranous particles, by exploiting their relatively low buoyant density. In some embodiments, size exclusion can be used to separate XOs from biochemically similar, but biophysically different MVs, which possess larger diameters of up to 1,000 nm. In some embodiments, differences in flotation velocity further allows for separation of differentially sized XOs. In general, as disclosed elsewhere herein, XO sizes will possess a diameter ranging from 30-200 nm, including sizes of 40-100 nm. In some embodiments, further purification can be performed based on specific properties of the particular XO of interest. In some embodiments, XOs (and/or EVs) can be further purified through, for example, the use of immunoadsorption with a protein of interest to select specific vesicles with exoplasmic or outward orientations.

Among current methods, for example, differential centrifugation, discontinuous density gradients, immunoaffinity, ultrafiltration and high performance liquid chromatography (HPLC), differential ultracentrifugation can be used for XO (and/or EV) isolation. In some embodiments, increasing centrifugal forces can be used. In some embodiments, centrifugal forces of greater than or equal to about 100000xg, about 10000xg, about 5000xg, about 2000xg, or ranges spanning and/or including the aforementioned values can be used to separate medium- and larger-sized particles and cell debris from the XO (and/or EV) pellets. Centrifugation alone allows for significant separation/collection of XOs (and/or EVs) from a conditioned medium. In some embodiments, enhanced specificity of XO (and/or EV) purification may employ sequential centrifugation in combination with ultrafiltration, or equilibrium density gradient centrifugation in a sucrose density gradient, to provide for the greater purity of the exosome preparation (flotation density 1.1-1.2g/mL) or application of a discrete sugar cushion in preparation. In some embodiments, separation is performed using flotation densities of greater than or at least about: 2 g/mL, 1.5 g/mL, 1.2 g/mL, 1.1 g/mL, 1.0 g/mL, 0.75 g/mL, or ranges spanning and/or including the aforementioned values.

As disclosed elsewhere herein, in some embodiments, ultrafiltration can be used to purify XOs, MVs, and/or EVs without compromising their biological activity. In some embodiments, membranes with different pore sizes can be used to separate XOs, MVs, and/or EVs from undesired particles - such as membranes with a molecular weight cut-off (MWCO) less than or equal to about: 200 kDa, 100 kDa, 75 kDa, 50 kDa, or ranges including and/or spanning the aforementioned values. In some embodiments, gel filtration can alternatively or also be used to eliminate smaller particles. In some embodiments, membrane (e.g., dialysis, ultrafiltration, etc.) and/or gel filtration is performed using a substantially physiological pH and/or at substantially physiological salt concentrations (e.g., avoiding the use of a nonneutral pH or non-physiological salt concentration). In some embodiments, tangential flow filtration (TFF) systems used. In some embodiments, TFF systems are scalable (to >10,000L), allowing one to not only purify, but concentrate the XO, MV, and/or EV fractions. In some embodiments, such approaches are advantageously less time consuming than differential centrifugation. In some embodiments, HPLC is used to purify the XOs, MVs, and/or EVs. In some embodiments, HPLC can also be used to purify XOs to homogeneously sized particles and preserve their biological activity as the preparation is maintained at a physiological pH and salt concentration.

In some embodiments, chemical methods are used to isolate XOs, MVs, and/or EVs. In some embodiments, these chemical methods include separation by differential solubility in precipitation techniques. In some embodiments, a precipitation reagent is added to a solution of XOs, MVs, and/or EVs to purify the XOs, MVs, and/or EVs. In some embodiments, these chemical methods include separation by addition to volume-excluding polymers (e.g., polyethylene glycols (PEGs), etc.). In some embodiments, these chemical methods can be combined with additional rounds of centrifugation or filtration, etc. In some embodiments, for example, a precipitation reagent, ExoQuick^{®}, is added to a conditioned cell media to quickly and rapidly precipitate a population of XOs. In some embodiments, flow field-flow fractionation (F1FFF) is an elution-based technique that is used to separate and characterize macromolecules (e.g., proteins) and nano- to micro-sized particles (e.g., organelles and cells) and which is successfully applied to fractionate XOs, MVs, and/or EVs from culture media.

In some embodiments, beyond the techniques disclosed elsewhere herein, relying on biochemical and biophysical features of XOs, MVs, and/or EVs, focused techniques may be applied to isolated specific XOs of interest. In some embodiments, antibody immunoaffinity is used to recognize XOs, MVs, and/or EVs associated antigens. In some embodiments, XOs, MVs, and/or EVs express the extracellular domain of membrane-bound receptors at the surface of the membrane of the parent cells. In some embodiments, this expression allows isolating and segregating XOs, MVs, and/or EVs in connections with their parental cellular origin, based on a shared antigenic profile. In some embodiments, conjugation to magnetic beads, chromatography matrices, plates or microfluidic devices, and/or combinations of such techniques with other techniques disclosed herein allows isolating of specific XO, MV, and/or EV populations of interest (e.g., as may be related to their production from a parent cell of interest or associated cellular regulatory state). Other affinity-capture methods use lectins which bind to specific saccharide residues on the EV surfaces (e.g., XOs and MVs).

Described herein are compositions and methods providing significant benefits in the treatment of cancer using CDCs, XOs, MVs, and/or EVs, including XOs such as CDC-derived XOs, MVs, and/or EVs and newt A1 cell line XOs, MVs, and/or EVs. Certain supporting techniques are described in, for example, U.S. App. No. 11/666,685, 12/622,143, 12/622,106, 14/421,355, PCT App. No. PCT/US2013/054732, PCT/US2015/053853, PCT/US2015/054301, PCT/US2016/035561, and PCT/US2018/028184.

In some embodiments, described herein is a method of treating cancer, including administering a therapeutically effective amount of a composition including CDCs, XOs, MVs, EVs, CDC-XOs, CDC-MVs, and/or CDC-EVs to a subject afflicted with cancer (or at risk of developing cancer), thereby treating the subject. In some embodiments, the administration is systemic. In other embodiments, the systemic administration is intraperitoneal injection. In some embodiments, the administration is local. In some embodiments, the administration is local and systemic. In some embodiments, the local administration is subcutaneous. In various embodiments, administration of the CDCs, XOs, MVs, EVs, CDC-XOs, CDC-MVs, and/or CDC-EVs includes administration of a therapeutically effective amount of the CDCs, XOs, MVs, EVs, CDC-XOs, CDC-MVs, and/or CDC-EVs. In various embodiments, the quantities (e.g., the number of particles) of CDCs, XOs, MVs, EVs, CDC-XOs, CDC-MVs, and/or CDC-EVs that are administered to achieved these effects range from 1 × 10⁶ to 1 × 10⁷, 1 × 10⁷ to 1 × 10⁸, 1 × 10⁸ to 1 × 10⁹, 1 × 10⁹ to 1 × 10¹⁰, 1 × 10¹⁰ to 1 × 10¹¹, 1 × 10¹¹ to 1 × 10¹², 1 × 10¹² or more. In other embodiments, the numbers of CDCs, XOs, MVs, EVs, CDC-XOs, CDC-MVs, and/or CDC-EVs is relative to the number of cells used in a clinically relevant dose for a cell-therapy method. In various embodiments, single doses are compared to two, three, four, four or more sequentially-applied doses. In some embodiments, as disclosed elsewhere herein, the EVs are obtained from cardiospheres, cardiosphere-derived cells (CDCs) or newt A1 cell line. In some embodiments, the EVs are XOs (e.g., CDC-XOs).

In some embodiments, a therapeutically effective amount of a composition includes about 1 × 10⁵ to about 1 × 10⁸ or more CDCs in a single dose. In another example, the number of administered CDCs includes 25 million CDCs per coronary artery (i.e., 75 million CDCs total) as another baseline for exosome dosage quantity. In various embodiments, the numbers of CDCs includes 1 × 10⁵, 1 × 10⁶, 1 × 10⁷, 1 × 10⁸, and/or 1 × 10⁹ in a single dose as another baseline for EV dosage quantity. In some embodiments, a therapeutically effective amount of CDCs includes less than or equal to about: 75 × 10⁶ CDCs, 150 × 10⁶ CDCs, 300 × 10⁶ CDCs, 400 × 10⁶ CDCs, 500 × 10⁶ CDCs, or ranges including and/or spanning the aforementioned values. In some embodiments, the quantities of EVs administered in each dose (where a single or multiple doses are used) and/or over the course of a treatment regimen ranges from 1 × 10⁶ to 1 × 10⁷, 1 × 10⁷ to 1 × 10⁸, 1 × 10⁸ to 1 × 10⁹, 1 × 10⁹ to 1 × 10¹⁰, 1 × 10¹⁰ to 1 × 10¹¹, 1 × 10¹¹ to 1 × 10¹², 1 × 10¹² or more. In certain instances, this may be prorated to body weight (range 100,000-1M CDCs/kg body weight total CDC dose). In various embodiments, the administration can be in repeated doses, such as two, three, four, four or more sequentially-applied doses.

In some embodiments, the number of EVs delivered to the subject in a dose (or dosing regimen) is determined based on the number of CDCs that would be used in a clinically effective dose in a cell-based therapy method. For example, in some embodiments, where 75-500 × 10⁶ CDCs is an effective dose for therapeutic treatment of cancer, using the equivalent amount of EVs that would be released by those CDCs in vivo would be administered to a patient in a cell-free method of treatment. In other words, CDC equivalent doses of EVs, MVs, XOs, CDC-EVs, CDC-MVs, and/or CDC-XOs can be used. As an illustration, in some embodiments, 3 mL / 3 × 10⁸ CDCs, is capable of providing therapeutic benefit. Therefore, a plurality of CDC-XOs as would be derived from that number of CDCs over the time course of those CDCs' residence in the body is used. In some embodiments, the amount of EVs, MVs, XOs, CDC-EVs, CDC-MVs, and/or CDC-XOs delivered to the patient is the amount of EVs, MVs, XOs, CDC-EVs, CDC-MVs, and/or CDC-XOs that would be released via an injection of equal to or at least about: 75 × 10⁶ CDCs, 150 × 10⁶ CDCs, 300 × 10⁶ CDCs, 400 × 10⁶ CDCs, 500 × 10⁶ CDCs, or ranges including and/or spanning the aforementioned values. In some embodiments, a dose of CDCs ranges between about 10 and 90 million CDCs, including about 10 to about 20 million, about 20 to about 30 million, about 30 to about 50 million, about 50 to about 60 million, about 60 to about 70 million, about 70 to about 75 million, about 75 million to about 80 million, about 80 million to about 90 million, and ranges including and/or spanning the aforementioned values. In several embodiments, the dose of CDCs ranges from about 30 million to about 1.5 billion CDCs, including about 30 million to about 45 million, about 40 million to about 50 million, about 50 million to about 50 million, about 60 to about 75 million, about 75 to about 1 billion, about 90 million to about 1.1 billion, about 1 billion to 1.25 billion, about 1.25 billion to about 1.5 billion, and ranges including and/or spanning the aforementioned values. In some embodiments, the equivalent amount of CDC-XOs or CDC-EVs delivered to the patient is calculated as the amount of CDC-XOs or CDC-EVs that would be released via an administration (e.g., injection or infusion) of the disclosed amounts CDCs over a given time of CDC residence in the body of about 1 week, about 2 weeks, about 3 weeks, or more. In certain instances, the dosage may be prorated to body weight (range 100,000-1M CDCs/kg body weight total CDC dose). In some embodiments, for injection into the heart, the number of administered CDCs includes 25 million CDCs per coronary artery (i.e., 75 million CDCs total) as another baseline for XO or EV dosage quantity.

In some embodiments, the CDC, XO, MV, EV, CDC-XO, CDC-MV, and/or CDC-EV quantity delivered to the patient (e.g., the dose) may be measured by weight (in mg) of CDCs, XOs, MVs, EVs, CDC-XOs, CDC-MVs, and/or CDC-EVs. For instance, in some embodiments, a dose of CDCs, XOs, MVs, EVs, CDC-XOs, CDC-MVs, and/or CDC-EVs may comprise equal to or at least about the following weights in mg: about 0.001 to about 0.005, about 0.005 to about 0.01, about 0.01 to about 0.05, about 0.05 to about 0.1, about 0.1 to about 0.5, about 0.5 to about 1, about 1 to about 10, about 10 to about 25, about 25 to about 50, about 50 to about 75, about 75 to about 100, or ranges including and/or spanning the aforementioned values. As discussed in additional detail herein, those masses are representative, of the number of CDCs, CDC-XOs or CDC-EVs that are dosed to a subject, depending on the embodiment. For example, in several embodiments, the number of CDCs in a dose can range from about 5 × 10⁴ to about 2 × 10⁹, including about 5 × 10⁴ to about 1 × 10⁵, about 1 × 10⁵ to about 2.5 × 10⁵, about 2.5 × 10⁵ to about 1 × 10⁶, about 1 × 10⁶ to about 1 × 10⁷, about 1 × 10⁷ to about 1 × 10⁸, about 1 × 10⁸ to about 1 × 10⁹, about 1 × 10⁹ to about 2 × 10⁹, about 2 × 10⁹ to about 5 × 10⁹, and ranges including and/or spanning the aforementioned values. Likewise, depending on the embodiment, the number of XOs or particles (e.g., vesicles) dosed to a subject can range from about 1 × 10⁹ to about 2 × 10¹⁴, including about 1 × 10⁹ to about 2 × 10⁹, about 2 × 10⁹ to about 4 × 10⁹, about 4 × 10⁹ to about 1 × 10¹⁰, about 1 × 10¹⁰ to about 1 × 10¹¹, about 1 × 10¹¹ to about 1 × 10¹², about 1 × 10¹² to about 2 × 10¹², about 2 × 10¹² to about 2 × 10¹³, about 2 × 10¹³ to about 1 × 10¹⁴, about 1 × 10¹⁴ to about 2 × 10¹⁴, and ranges including and/or spanning the aforementioned values. In some embodiments, the CDC, XO, MV, EV, CDC-XO, CDC-MV, and/or CDC-EV quantity delivered to the patient may be measured by protein weight (in mg) and/or by total cell or vesicle weight (e.g., where water has been removed from the area outside the cells or vesicles). In some embodiments, the CDC, CDC-XO, and/or CDC-EV quantity delivered to the patient is equal to 1-10, 10-25, 25-50, 50-75, 75-100, or 100 or more mg protein. In some embodiments, administering a therapeutically effective amount of a composition includes about 1 to about 100 mg XO and/or EV protein in a single dose.

In some embodiments, the administration of CDCs, XOs, MVs, EVs, CDC-XOs, CDC-MVs, and/or CDC-EVs to a subject in need thereof includes a single dose and/or multiple doses (e.g., 2, 4, 6, 8, 10, or more doses). In some embodiments, where multiple doses are used, the administration of CDCs, XOs, MVs, EVs, CDC-XOs, CDC-MVs, and/or CDC-EVs is performed daily, weekly, biweekly, every three weeks, monthly, every six months, or every year. In some embodiments, the dosing schedule is performed over a period of, for example, 2 weeks, 1 month, 2 months, 3 months, 5 months, 6 months, a year, 5 years, or ranges including and/or spanning the aforementioned values. For illustration, in some embodiments, the interval includes the administration of 2-10 doses at intervals of 1-5 months. In some embodiments, the dosing schedule is 3 doses with about 3 months between each dose. In some embodiments, the dosing schedule is 5 doses with about 1 week separating each dose. In some embodiments, the dosing schedule is 3 administrations (e.g., 3 single doses at different times) at weeks 0, 6 and 9. In some embodiments, an interval schedule is used, where there are periods of dosing and periods of rest between dosing periods (e.g., weekly doses for a month followed by a rest period of 5 months, followed by weekly doses for a month and so on). In some embodiments, a single dose comprises a therapeutically effective amount of CDCs, XOs, MVs, EVs, CDC-XOs, CDC-MVs, and/or CDC-EVs. In some embodiments, the dosing periods and/or interval schedule is performed throughout the life of the patient. In some embodiments, multiple administrations of each single dose are provided to the subject. In various embodiments, as disclosed elsewhere herein, the administration can be in repeated doses, such as two, three, four, four or more sequentially-applied doses.

In some embodiments, administration of the therapeutically effective amount in a dosage regime depends on the subject to be treated. In some embodiments, administration in a dosage regime may be a single dose, or multiple administrations of dosages over a period of time spanning 10, 20, 30, 40, 50, 60 minutes, and/or 1, 2, 3, 4, 5, 6, 7, 8, 9, 10, 11, 12, 13, 14, 15, 16, 17, 18, 19, 20, 21, 22, 23, 24 or more hours, and/or 1, 2, 3, 4, 5, 6, 7, days or more. Moreover, administration may be through a time release or sustained release mechanism, implemented by formulation and/or mode of administration.

In some embodiments, treating cancer in the subject includes a reduction in tumor weight. In some embodiments, treating cancer in the subject includes a reduction in tumor vascularization. In some embodiments, treating cancer in the subject includes a reduction in tumor invasion, metastasis, or both. In some embodiments, treating cancer in the subject includes a decrease in serum lactate dehydrogenase. In some embodiments, treating cancer in the subject includes a decrease in expression of ERBB2/ERBB3, MDM4, IGF1R and/or IRF5. In some embodiments, treating cancer in the subject includes a decrease in TERT and/or telomerase enzymatic activity.

Also described herein is a method of preventing cancer, including administering a composition including CDCs, XOs, MVs, EVs, CDC-XOs, CDC-MVs, and/or CDC-EVs to a subject susceptible to cancer. In some embodiments, the subject possess one or more genetic mutations (e.g., genetic mutations that predispose the subject to a cancer). In some embodiments, the administration is systemic. In some embodiments, the systemic administration is intraperitoneal injection. In some embodiments, the administration is local. In some embodiments, the local administration is subcutaneous. In some embodiments, the XOs, MVs, EVs, CDC-XOs, CDC-MVs, and/or CDC-EVs are obtained from cardiospheres, CDCs, and/or newt A1 cell line. In other embodiments, the EVs are XOs (e.g., CDC-XOs).

Further described herein is a method of treating cancer, including administering a therapeutically effective amount of a composition including CDCs, XOs, MVs, EVs, CDC-XOs, CDC-MVs, and/or CDC-EVs to a subject afflicted with cancer, thereby treating the subject. In some embodiments, the administration is systemic. In various embodiments, administration of the CDCs, XOs, MVs, EVs, CDC-XOs, CDC-MVs, and/or CDC-EVs includes administration of a therapeutically effective amount of the CDCs, XOs, MVs, EVs, CDC-XOs, CDC-MVs, and/or CDC-EVs. In some embodiments, administering a composition includes injection. In some embodiments, treating cancer in the subject includes a reduction in tumor weight. In some embodiments, treating cancer in the subject includes a reduction in tumor vascularization. In some embodiments, treating cancer in the subject includes a reduction in tumor invasion, metastasis, or both. In some embodiments, treating cancer in the subject includes a decrease in serum lactate dehydrogenase.

In some embodiments, the methods of treatment comprise selecting a subject in need of treatment. In some embodiments, the methods of treatment comprise administering to the subject (e.g., a subject suffering from cancer or a disease state associated therewith or a patient susceptible to cancer) a therapeutically effective amount of CDCs, XOs, MVs, EVs, CDC-XOs, CDC-MVs, and/or CDC-EVs. In some embodiments, the CDCs, XOs, MVs, EVs, CDC-XOs, CDC-MVs, and/or CDC-EVs are autologous or allogeneic to the subject (e.g., derived from their own tissue, from another subject's tissue, and/or from the tissue of another animal species). In some embodiments, the methods of treatment comprise administering to the subject a therapeutically effective amount of molecular cargo from XOs, MVs, EVs, CDC-XOs, CDC-MVs, and/or CDC-EVs. In some embodiments, molecular cargo of CDC-XOs or CDC-EVs is isolated and/or synthesized and that molecular cargo (e.g., particular molecules and/or combinations of different molecules, including RNA polynucleotides and/or short non-coding RNAs) is administered to the subject in need thereof (e.g., a subject suffering from cancer or a disease state associated therewith or a patient susceptible to cancer). In some embodiments, the method of treatment comprises administering to the subject a therapeutically effective amount of an isolated RNA polynucleotide or a vector encoding (and/or containing) a RNA polynucleotide found in XOs, MVs, EVs, CDC-XOs, CDC-MVs, and/or CDC-EVs.

In some embodiments, the CDCs, XOs, MVs, EVs, CDC-XOs, CDC-MVs, and/or CDC-EVs are delivered to the subject systemically. In some embodiments, the XOs, MVs, EVs, CDC-XOs, CDC-MVs, and/or CDC-EVs are delivered to the subject systemically and locally. In some embodiments, the CDCs, XOs, MVs, EVs, CDC-XOs, CDC-MVs, and/or CDC-EVs are delivered to the subject systemically but not locally. In some embodiments, the CDCs, XOs, MVs, EVs, CDC-XOs, CDC-MVs, and/or CDC-EVs are delivered to the subject systemically locally. In some embodiments, the XOs, MVs, EVs, CDC-XOs, CDC-MVs, and/or CDC-EVs are delivered to the subject locally but not systemically. In some embodiments, non-limiting examples of a methods to administer a therapeutically effective amount of CDCs, XOs, MVs, EVs, CDC-XOs, CDC-MVs, and/or CDC-EVs include systemic administration (e.g., intravenous, intra-arterial, intraventricular, intra-aortic, and/or intraperitoneal injection and/or infusion). In some embodiments, the CDCs, XOs, MVs, EVs, CDC-XOs, CDC-MVs, and/or CDC-EVs are injected or infused intravenously. In some embodiments, a therapeutically effective amount of CDCs, XOs, MVs, EVs, CDC-XOs, CDC-MVs, and/or CDC-EVs is administered to a patient by intramuscular injection and/or infusion. In some embodiments, a therapeutically effective amount of CDCs, XOs, MVs, EVs, CDC-XOs, CDC-MVs, and/or CDC-EVs is administered to a patient by infusion directly at a local site (e.g., into or near a tumor and/or a target site where treatment is desired). In some embodiments, an effective amount of CDCs, XOs, MVs, EVs, CDC-XOs, CDC-MVs, and/or CDC-EVs is delivered systemically via injection and/or infusion at an area of the body that is not in the heart. In some embodiments, the intravenous administration of CDCs, XOs, MVs, EVs, CDC-XOs, CDC-MVs, and/or CDC-EVs includes jugular and/or femoral vein injection and/or infusion.

In some embodiments, as disclosed elsewhere herein, the method involves the administration of the molecular cargo of EVs to a patient. In some embodiments, the molecular cargo comprises an RNA polynucleotide and/or one or more RNA polynucleotides, such as 2, 3, 4, 5, 6, 7, 8, 9, 10 or more RNA polynucleotides. In some embodiments, the RNAs include non-coding RNAs. In some embodiments, the non-coding RNAs include tRNAs, yRNAs, rTNAs, mirRNAs, lcRNAs, piRNAs, snRNAs, snoRNAs, further including fragments thereof, among others. In some embodiments, the one or more RNA polynucleotides comprise microRNAs. In some embodiments, the microRNAs are selected from the group consisting of miR-146, miR-124, miR-210, miR-92, miR-320, miR-222, miR-223, miR-148a, miR-215, miR-33a, miR 204, miR-376c, miR4532, miR-4742, miR-582, miR-629, miR-223, miR-3125, miR-3677, miR-376b, miR-4449, miR-4773, miR-4787, miR-491, miR-495, miR-500a, miR-548ah, miR-550, miR-548ah, miR-550a, miR-551n, miR-5581, and/or miR-616. In some embodiments, the microRNAs are selected from the group consisting of: microRNAs miR-146a, miR-124, miR-210, miR-92, miR-320, miR148a, miR-22, miR-24, miR-210, miR-150, miR-140-3p, miR-19a, miR-27b, miR-19b, miR-27a, miR-376c, miR-128, miR-320a, miR-143, miR-21, miR-130a, miR-9, miR-185, and miR-23a. In some embodiments, the microRNA includes miR-148a-3p. In some embodiments, the microRNAs are from the miR-17-92 cluster.

In some embodiments, one or more of the micro RNAs disclosed herein provide anti-cancer effects by downregulating one or more cancer genes. In some embodiments, the method of treating cancer disclosed herein includes down regulating HIF-1α , IL-6, and/or EGF-R. In some embodiments, the methods disclosed herein do not significantly or substantially upregulate cathepsin D, eNOS, EpCAM, and/or ANGPTL4 in a way that would allow cancer proliferation.

In some embodiments, an effective amount of RNA ranges between 0.1 and 20 mg/kg, and/or 0.5 and 10 mg/kg (where kg is the weight of the patient). In some embodiments, the therapeutically effective amount is a single unit dose. In some embodiments, an effective amount of RNA includes concentration at a range between 0.1 nM and 10M. In some embodiments, the concentration of RNA ranges between 0.3 to 400 nM, or between 1 to 200 nM. In some embodiments, the RNA polynucleotide or vector includes concentrations that refer to the total concentration of RNA polynucleotide or vector added. In some embodiments, an amount of a RNA polynucleotide or vector is provided to a cell or organism is an effective amount for a particular result, which refers to an amount needed to achieve a desired goal, such as inducing a particular cellular characteristic(s). In some embodiments, an effective amount of RNA polynucleotide includes an amount capable of treating cancer.

In some embodiments, a formulation or a composition comprising CDCs, CDC-XOs, CDC-MVs, CDC-EVs, CDCs, XOs, MVs, EVs, molecular cargo of EVs is provided. In some embodiments, the formulation and/or composition includes a pharmaceutically acceptable carrier and/or excipient. In some embodiments, the carrier is water at physiologic pH and/or isotonicity.

### EXAMPLES

### Example 1

### Materials and Methods

### Cell lines

HT1080 human fibrosarcoma and NCI-H23 human neuroblastoma cells were purchased from ATCC. Human bone marrow-derived MSCs were purchased from Lonza. Cells were cultured per manufacturer's instructions. CDCs from human donors and rat hearts were derived as described in Example 2.

### Invasion assay

Invasion assays were done using a Cytoselect 24 well Cell Invasion Assay (colorimetric) kit (Cell Biolabs). An aliquot of the suspension was added to inserts coated with a uniform layer of reconstituted basement membrane proteins (ECMatrix). Minimal Essential Medium containing 10 % FBS was added to the lower chamber in which the coated inserts were placed. At 48 hours of incubation, the cells that had not invaded the membrane were washed away, and the invasive cells were stained with Cell stain solution. The dye from the stained cells was then extracted, and the color intensity was measured at OD 560 nm using a microplate reader.

### Adhesion assay

Cell adhesion potential was tested using CytoSelect^{™} 48-well Cell Adhesion Assay (ECM Array, Colorimetric Format). An equal number of cells was added to each Fibrinogen-coated well. After 1.5 hours of incubation, the plate was rinsed with PBS to discard the unattached cells, and the attached cells were stained with a cell stain solution. After extracting the dye from attached cells, the color intensity was measured at OD 560 nm using a microplate reader.

### Telomerase activity assay

Telomerase activity was determined using TeloTAGGG Telomerase PCR ELISAPLUS kit (Sigma-Aldrich). Cells were lysed with the mixture of lysis reagent and HaltTM protease inhibitor (Thermo Fisher Scientific) and checked for protein concentration using (Bio-Rad Quick StartTM). The cell extract was heated for 10 min at +85°C to create negative controls for each sample. One ug of total protein was used from each sample for combined primer elongation (telomeric repeat amplification protocol [TRAP reaction])/amplification (PCR) reaction. PCR products were then denatured and hybridized with digoxigenin (DIG)-labeled detection probes which is specific for telomeric repeats. The biotin-labeled primer was then used to immobilize the resulting product to a streptavidin-coated microplate, which was then detected with an anti-digoxigenin antibody conjugated with horseradish peroxidase and TMB substrate. The plate was then read twice with a microplate reader at OD 450 nm.

### RT-PCR Array

RNA was extracted from HT1080 cells using RNeasy Plus mini kit (QIAGEN) and from tumor tissue using RNeasy plus universal kit (QIAGEN). For further purification of RNA, the RNase-Free DNase set (Qiagen) was used, and the RNA integrity was assessed by Nano drop. cDNA was generated from the purified RNA using RT2 first strand kit. PCR was performed on Applied bio system 96 well fast block cycler using RT2 Profiler PCR Array human cancer drug target and RT2 Real-Time SYBR Green PCR Master Mix (Qiagen). The array evaluated the expression of 84 cancer marker genes and five housekeeping genes for data normalization. Gene expression was then amplified over the course of 40 cycles and analyzed by ddCt method.

### Proteome profiler assay

Tumor tissues were lysed using a mixture of RIPA lysis extraction buffer and HaltTM protease inhibitor (Thermo Fisher Scientific), and cells were lysed using proteome profiler lysis buffer 17. Lysed cells and tumor tissue were incubated overnight with Human Phospho-Kinase Array nitrocellulose membranes which were spotted with different capture and control antibodies. Unbounded proteins were washed away, and the array was then incubated with a cocktail of biotinylated detection antibodies. After application of Streptavidin-HRP and chemiluminescent detection reagents, a signal was produced at each capture spot based on the amount of bounded phosphorylated protein. The average signal (pixel density) of the pair of duplicate spots representing each protein was determined after subtracting an averaged background signal.

### LDH assay

Serum for LDH assay use was obtained from blood samples collected on day 18 and 25 from treated and untreated mice. After adding the master mix to the biological samples, the LDH activity was measured when LDH reduced NAD to NADH, which then interacted with a particular probe to produce a color. The output was then measured every 3 minutes at OD 450 on a light protected kinetic microplate reader at 37°C for 30 minutes.

### Inmmunostaining

Tissue slices were washed with PBS and fixed with 4% paraformaldehyde followed by blocking solution (Dako). Slices were incubated overnight at 4°C with the primary antibody. Ki-67 and CD-31 antibodies were purchased from Abcam. Slices were washed 3 times with PBS and incubated with a secondary antibody of the appropriate species and *in situ* cell death detection kit, fluorescein (Roche). All slides were counterstained for DAPI (Sigma). Five to 10 images per slide were imaged at x20 magnification using a confocal laser microscope and analyzed using Image-J software.

### Animal models

Two different animal models were used: human xenograft fibrosarcoma in nude athymic Foxn1nu mice, and spontaneous acute lymphocyte leukemia (ALL) in 2-year old Fisher 344 rats. The ALL, also denoted as large granular lymphocyte (LGL) leukemia, is one of the leading causes of death in old F344 rats. The phenotype of the leukemic cells resembles that of human NK-LGL leukemia. All animal procedures were conducted in accordance with humane animal care standards outlined in the NIH Guide for the Care and Use of Experimental Animals and were approved by the Cedars-Sinai Animal Care and Use Committee.

### MicroRNA array analysis

To characterize the microRNA (miR) cargo of CDC- and MSC-derived EVs, miR was extracted using the miRNeasy kit (Qiagen) and analyzed with MiScript array with a total of 88 genes (Qiagen), according to manufacturer's instructions.

### Statistical analysis

All results are presented as mean ± SEM or percentages, for continuous and categorical variables, respectively. Significance of differences was assessed by Student t test or 1-way ANOVA in cases of multiple groups if the distribution of the variable was normal; otherwise, the Mann-Whitney or Kruskal-Wallis tests were used. Tumor volume data were tested across treatment groups with mixed-model regression to account for the repeated measures within each animal. Post-hoc testing was adjusted for multiple comparisons (Tukey). Data was log-transformed prior to analysis and residuals were inspected to confirm data met assumptions necessary for parametric assessment. For survival analysis Breslow-Wilcoxon test was applied to compare leukemia-free survival curves. All probability values reported are 2-sided, with p<0.05 considered significant. IBM SPSS Statistics 20 and SAS v9.4 were used for all analyses. For in vitro studies the lowest number of replicates per experiment was three.

### Example 2

### Heart Cell Isolation and Culturing

When minced heart tissue is grown in primary culture, it gives rise to monolayers of cardiac stromal cells and progenitor cells known as explant-derived cells (EDCs). Such EDCs are the precursors to cardiospheres and CDCs. Representative methods for EDC and CDC isolation and culture can be found in U.S. Application Publication No. 2012/0315252. In brief, myocardial biopsies from healthy hearts of deceased tissue donors (for human tissue) were minced into small fragments, digested with collagenase, and cultured on fibronectin-coated dishes. EDCs grew spontaneously from the tissue fragments and reached confluence by 2-3 weeks, at which time they were harvested using 0.25% trypsin (GIBCO), purified from tissue and cell debris and re-plated. EDCs were cultured in suspension on 20 µg/ml poly d-lysine (BD Biosciences) to form self-aggregating cardiospheres. CDCs were obtained by seeding cardiospheres onto fibronectin-coated dishes and passaged 2-4 times. Cultures were maintained in 5% CO2 at 37°C, using IMDM basic medium (GIBCO) supplemented with 20% FBS (Hyclone), 1% penicillin/streptomycin, and 0.1 ml 2-mercaptoethanol. All protocols were approved by the institutional review board for human and animal subjects research.

### Isolation and Characterization of Extracellular Vesicles

EVs were harvested from serum-free condition media conditioned for 15 days by passage 3 CDCs and bone marrow-derived mesenchymal stem cells (MSCs). Media was then subjected to two successive centrifugation steps to remove cellular debris: 2,000xg for 20 min and 10,000xg for 30 min. The resulting supernatant was precipitated by polyethylene glycol (ExoQuickTC), which yielded high quantities of purified XOs, followed by overnight incubation at 4°C. EVs were then isolated by centrifugation at 2,000xg for 30 min, re-suspended in phosphate buffered saline (PBS) and quantified for particle concentration and size using the LM10-HS system (NanoSight), and protein concentration (BQuick StartTM).

### Example 3

### In Vitro Studies

EVs secreted by cardiosphere-derived cells (CDCs, heart progenitor cells) were tested in vitro on fibrosarcoma HT1080 and neuroblastoma NCl-H23 cells. HT1080 or NCl-H23 cells were counted manually using the Neubauer Ruled hemocytometer and reconfirmed with a TC20^{™} Automated Cell Counter. Viability was checked by trypan blue staining. Equal numbers of viable cells were plated in 6 or 12-well plates; after an initial 24-hour stabilization period in minimal essential medium, Eagle's minimal essential medium, and Dulbecco's Modified Eagle's Medium (Thermo Fisher Scientific) fetal-bovine-serum (10%) supplemented medium, the cells were washed with PBS and then incubated with serum-free medium (SF) alone or containing re-suspended CDC-EVs (1 mg of EV-protein per 106 cells; Figure 7A).

After 96 or 120 hours the cells were harvested, washed in PBS and used for various assays. HT1080 or NCl-H23 were incubated in serum-free medium (SF) alone or SF containing re-suspended CDC-EVs (1 mg of EV-protein per 10⁶ cells; Figure 7A).

Global CDC-EV-induced changes in expression of cancer-related proteins and genes were characterized using specific arrays, rather than focusing on a single pathway. After HT1080 cells were incubated for 96 hours with CDC-EVs or SF medium alone in vitro (Figure 1A), significant differences were observed in 11 of 84 proteins analyzed (Figure 7A). Although not unidirectional, most of the observed differences (downregulation of proteins such as enolase 2, c-Met, mesothelin, PDGF-AA, eNOS, IL-6, and upregulation of CA125) suggested a negative impact of CDC-EVs on pathways associated with cancer. Negative effects were further confirmed as CDC-EV-primed HT1080 cells showed lower invasion and adhesion properties compared with cells incubated with SF medium alone (Figure 1B, C) and CDC-EV-primed NCl-H23 cells were less viable after 96 hours compared with the NCl-H23 cells incubated in SF media only (Figure 7B). Cancer drug target transcripts (n=84) were likewise quantified in HT1080 cells with or without CDC-EV priming. Thirty-five genes were significantly up or down-regulated (Figure 7C); those with at least two-fold changes between groups are shown in Figure 1D. The most down-regulated was the TERT gene, coding for the catalytic subunit of telomerase. A marked decrease of telomerase enzymatic activity (Figure 1E) was noted under the same conditions.

### Example 4: In Vivo Studies

In vivo models comprised the xenograft HT1080 fibrosarcoma in athymic mice (n=35), and spontaneous acute lymphocyte leukemia in old rats (n=44). CDC-EVs were compared with two control groups: EVs secreted by bone-marrow derived mesenchymal stem cells (MSC-EVs) and phosphate-buffered saline (PBS).

### Athymic Mice Study

To create xenograft tumors, 10⁶ HT1080 cells, re-suspended in PBS, were injected subcutaneously into the right and left flanks of nude athymic mice. The animals were then subjected either of two different protocols based on the EV-delivery method and the dose. In the systemic-delivery protocol using intraperitoneal (i.p.) administration 24-hours after HT1080 cell injection, mice were randomly allocated among three groups: human-CDC-EVs (n=6) (CDC-EV hereinafter, unless host specified), human-MSC-EVs (n=8), or PBS alone (n=9). EVs (1mg protein) were re-suspended in 1mL of PBS or 1mL PBS alone was used and repeated after 2 weeks. In the local-delivery protocol, using peri-tumoral subcutaneous (s.c.) administration of treatment 24-hours after HT 1080 cell injection, mice were randomly allocated to either of two groups: rat-CDC-EVs (n=6) or PBS alone (n=6). EVs (2mg protein) were re-suspended in 1mL of PBS, or 1mL PBS alone, was injected locally and repeated on a weekly basis.

### Acute Lyphocyte Leukemia (ALL) Study

In the rat model of spontaneous acute lymphocyte leukemia (ALL), after initial functional evaluation, a total of 44 animals (males and females) were allocated in two groups, ensuring similar baseline characteristics. Twenty-four rats received a dose of 7 µg-EV protein/gr body weight of rat-CDC-EV via systemic intra-arterial (i.a.) injection. The remaining (n=20) rats, in the control group, received i.a. PBS monthly during the 4-month follow-up period. The diagnosis of ALL was established when the clinical picture was associated with either typical histopathological findings in the spleen and/or an abnormal peripheral blood test. Clinically, affected rats exhibited progressive decreases of exercise capacity, weight loss, pale eyes and jaundice. Splenomegaly was a constant finding in these rats. Histological findings included diffuse infiltration of the splenic red pulp with the neoplastic LGL; peripheral blood was characterized by marked leukocytosis (> 50^{x}103/µL, upper limit of normal 11^{x}103/µL) with atypical lymphocytosis (LGL). Regenerative anemia, thrombocytopenia and abnormal liver function tests were common findings, and death usually occurred within 2-3 weeks of the first clinical signs.

### Testing

To measure the tumor growth in the mice fibrosarcoma model, the two longest perpendicular axes in the x/y plane of each xenograft tumor were measured periodically, using a digital Vernier caliper. The tumor volume was calculated according to equation Vol=a^{x}b2/2. Food-water consumption and body weight were also measured, to monitor for cachexia. Blood samples were obtained on days 18 and 25. The animals were followed-up for 30 days, euthanized, and tumors and lungs were harvested for further analysis.

The impact of CDC-EVs in vivo was tested. In mice with a xenograft fibrosarcoma, both systemic and local treatment with human- or rat-CDC-EVs (Figure 2A, B) was associated with ~2.4-fold decrease (p<0.01 and p<0.05 for human and rat, respectively) of tumor growth compared with PBS mice (Figure 2C-F). Mean tumor weight was 1.5±0.3 gr and the proportion of mice with a tumor weight > 1.5 gr was 62.5% in the PBS group compared with 16.6% in the rat-CDC-EV-treated animals (p<0.05; Figure 2G). Decreased tumor growth was associated with 2-fold reduction of tumor cell proliferation measured by expression of Ki67 (p<0.001) and 1.5-fold increase of apoptosis based on terminal deoxynucleotidyl transferase dUTP nick end labeling (p<0.05) in CDC-EV vs PBS mice (Figure 3A, B). CDC-EVs decrease fibrosarcoma growth by decreasing proliferation and increasing apoptosis of tumor cells in mice.

Injection of CDC-EVs led to a 2.5-fold decrease of fibrosarcoma growth in mice (p<0.01 and p<0.05 for human and rat EVs, respectively) vs PBS group. The effect was associated with 2-fold decrease of tumor cells proliferation (p<0.001) and 1.5-fold increase of apoptosis (p<0.05) in CDC-EV vs PBS mice. Salutary changes in tumor gene and protein expression were observed in CDC-EV animals. CDC-EVs reduced tumor vascularization compared with PBS (p<0.05) and MSC-EVs (p<0.01). Moreover, CDC-EVs increased leukemia-free survival (p<0.05) in old rats vs PBS. MiR-146, highly enriched in CDC-EVs, may be implicated in part of the observed effects.

Bone marrow-derived mesenchymal stem cells (MSC) are commonly used in regenerative medicine trials, but their safety in cancer is controversial. Given this concern the MSC-derived EVs (MSC-EV) were used as a second comparator group for the CDC-EV treated group of mice (Figure 2A). Unlike CDC-EV associated decrease of the tumor volume, MSC-EV treated mice did not present significant differences in the external tumor growth compared with PBS (Figure 2C, E, F), and animals in the MSC-EV group had higher tumor weight compared with rat-CDC-EV treated mice (p<0.05; Figure 2G).MSC-EVs increase metastatic spread of cancer cells with related increased tumor vascularization compared with CDC-EV.

Although tumor cell proliferation was lower in the MSC-EV treated mice than in the PBS group (p<0.01), it was higher than in CDC-EV treated animals (p<0.05; Figure 3A, B). No significant effect was observed on apoptosis (Figure 3A, B). Analysis of the gene expression pattern revealed some directionally-opposite effects in MSC-EV vs CDC-EV treated mice, as compared to the differences between CDC-EV and PBS animals (Figure 3E). In the latter case, most of the genes were downregulated, but MSC-EV mice had higher expression levels of cathepsins (CTSS and CTSD) and growth factors such as ERBB4, ERBB2, FIGF, EGFR compared to CDC-EV treated mice (Figure 8C). Curiously, telomerase (TERT) expression was also 1.6-fold higher (p<0.01) in the MSC-EV vs CDC-EV groups. Other differences such as markedly upregulated expression of PTGS2 (3.8-fold; p<0.00001) and HDAC11 (3.1-fold; p<0.001) in MSC-EV vs CDC-EV animals, may reflect higher pro-inflammatory properties of the former particles.

Similarly to the gene expression, differences in tumor protein levels in MSC-EV treated mice were opposite to those observed in CDC-EV injected animals for roughly half the proteins probed (Figure 4A). While downregulated in CDC-EV vs PBS groups, levels of cathepsin D, eNOS, EpCAM and ANGPTL4 were up-regulated in MSC-EV vs CDC-EV treated mice. All promote tumor development by increased angiogenesis (VEGF expression was also significantly higher in the MSC-EV group) and/or invasiveness of tumor cells. Higher vascularization of the tumor in MSC-EV compared with the CDC-EV group of mice was also confirmed by CD31 staining (p<0.01; Figure 4B,C).

To analyze the metastatic spread of cancer cells, the human Y-RNA fragments in mouse lung tissue was measured with q-PCR. With high-moderate expression (Ct values <30 for almost all animals; Figure 9B), 6-fold higher levels of HT1080 fibrosarcoma cells in the lungs of MSC-EV treated mice vs PBS or CDC-EV groups was found (p<0.05 for all comparisons; Figure 4D). Although not specific, changes in serum levels of lactate dehydrogenase (LDH) was tested, in an attempt to assess the systemic impact of the cancer. High LDH levels are associated with an increased risk of death from prostate, pulmonary, colorectal, gastro-esophageal, gynecological and hematological cancers and changes in LDH levels during treatment may also predict overall survival in patients with metastatic cancer. A moderate increase of serum LDH levels in MSC-EV treated mice was found, unlike the marked decrease observed in the rat-CDC-EV treated group (p<0.05; Figure 4E). CDC-EV decrease spontaneous leukemia-related mortality in old rats

In the process of studying the rejuvenating effects of CDC-EVs in old rats, an effect of CDC-EVs on spontaneous acute leukemia, which is known to be prevalent and fatal in senescent rats, was noted. Animals treated with rat-CDC-EVs less frequently developed clinically overt ALL (characterized by jaundice, ~4-fold increase of spleen size and abnormal blood counts), than did PBS rats (12. 5% vs 30%, p=n.s.) (Figure 5A). Mean leukemia-free survival also increased from 107±11 days in the PBS group to 124±4 days in rats treated with CDC-EVs (p<0.05; Figure 5B). The latency for the development of advanced disease and death was increased two-fold in the CDC-EV group vs PBS (p<0.05; Figure 5C).

Only 3 of 24 rats in the CDC-EV arm developed clinical ALL. In one of the rats, once the diagnosis was confirmed by blood counting, an additional, double dose of rat-CDC-EVs was administered to evaluate the effect on cancer cells. After one week, the total number of white blood cells decreased from 82. 6×103/µL to 34. 6×103/µL, the absolute number of lymphocytes from 61124/ µL to 19722/µL, the 12390/µL neutrophils remained almost unchanged and monocytes increased from 1652/µL to 2768/µL, resulting in a change of the proportional distribution of the blood cells (Figure 5D), suggestive of an anti-leukemic effect.

Differential miR signature of EVs as a potential contributor to the anti-cancer effects of CDC-EVs were tested. CDC-EVs carry and transfer a diverse cargo including proteins, lipids and nucleic acids. MiRs, small regulatory RNA molecules stably transported by EVs, influence the expression of >60% of human protein-coding genes. MiRs affect the hallmarks of cancer, including sustaining proliferative signaling, evading growth suppressors, resisting cell death, activating invasion and metastasis, and inducing angiogenesis.

Based on this evidence and the oncogenic differences observed between CDC- and MSC-EVs, the EVs' miR cargo was investigated. First, it was observed that the same passage and number of initially-plated cells, obtained from human donors of similar age, differ in their EV production: CDCs secreted ~50% more EVs than did MSCs, and the mean size of the particles was ~50 nm smaller (Figure 6A). This may be related to higher secretion of XOs, which are the smallest EVs, by CDCs. Next, in comparing EV miRs (Figure 6B), it was observed that miR-146a was exclusive for human CDC-EVs and miR-92a was exclusive for human and rat CDC-EVs among the most abundant miRs. Globally (among abundant and non-abundant miRs), miR-146a was 87-fold up-regulated in human CDC-EVs compared with MSC-EVs (p<0.01; Figure 6C) and only 6. 2-fold higher compared with rat-CDC-EVs. Other miRs which were more abundant in CDC-EVs vs MSC-EVs included miR-124, miR-210, miR-92 and miR-320.

### Example 5

### Genomic and Proteomic Studies

To further probe the mechanisms underlying the observed anti-cancer effects of CDC-EVs, genomic and proteomic studies of the tumor were performed using the same arrays as for in vitro studies. Interestingly the pattern of gene expression differences between control mice and both CDC-EV groups were similar (systemic human [Figure 3C] and local rat [Figure 8Ai]), indicating a clear negative effect of CDC-EVs regardless of the EV species of origin or the delivery method. A generalized downregulation of cancer drug target genes was observed in the CDC-EV groups which reached statistical significance in 38% of the transcripts quantified (Figure 8B). Most of the downregulated genes were growth factors and their receptors, and transcription factors (Figure 3D). Comparing the in vivo and in vitro results, it was observed that, while downregulation of ERBB2/ERBB3, MDM4, IGF1R or IRF5 may be direct effects of CDC-EVs on the cancer cells, many others (such as a reduced expression of HIF1A, CTSL, TOP2A or PLK2) are indirect, host cell-mediated effects with a final negative impact on tumor growth.

Differences in tumor protein levels were measured. Of 84 analyzed proteins, 30 showed significant modulation with CDC-EV treatment compared with PBS treated animals (Figure 4A). In 87% of cases, the proteins belonged to pathways related to local tumor progression, metastasis and/or angiogenesis (i.e. cathepsin D, MMP-9, eNOS, Leptin, ANGPTL4, autotaxin). The endothelial cell marker CD31 (Figure 4B) was measured and it was observed that significantly lower vascularization of CDC-EV treated mice tumors compared with PBS group (p<0.05; Figure 4C).

### Example 6

### Discussion

It was demonstrated that a reduction of proliferation and an increase of programmed death of tumor cells, together with a prolongation of host survival in two different cancer types and different rodent species, associated with the use of human- and rat-CDC-EVs. Parent CDCs have been tested in different pre-clinical and clinical studies for therapeutic applications and have been demonstrated to have many favorable effects and no safety issues to date. In the field of oncology, a class of drugs that efficiently eliminates all cancer cells with no or minimal toxicity for normal cells is still not available. So, the relevance of the findings that CDCs and CDC-EVs are a non-toxic anticancer treatment approach is valuable.

It was observed that the reduction of tumor growth in CDC-EV treated animals was associated with a wide, local modulation of genomic and proteomic profiles, consistent with the diversity of bioactive components within EVs. Underlying the inhibition of tumor progression were CDC-EV-induced down-regulation of growth factors and their receptors, decreased levels of transcription factors, and a marked anti-angiogenic effect. The last effect provides evidence towards efficacy not only for the local development of the cancer but for metastatic cancer as well. The amplification (in terms of a higher number of modulated genes and proteins) of the CDC-EV induced anticancer effect in vivo compared with in vitro studies, suggests that part of the effect is mediated indirectly, potentially through reconditioning of different host cells. Tumor microenvironment is known to be essential for sustained cancer growth, invasion and metastasis and tumor stromal cells have been proposed as an attractive therapeutic target. CDCs and CDC-EVs were demonstrated to modulate fibroblasts and macrophages, both cell types with relevance to the tumor microenvironment. CDCs were also demonstrated to reduce tissue myofibroblast infiltration, which release metalloproteinases (MMP) and lead to extracellular matrix (ECM) remodeling and the liberation of growth factors embedded in the ECM, tumor growth, local invasion and vascularization. Activated tumor-associated macrophages secrete G-CSF, IL-6 and VEGF, promoting angiogenesis and creating an inflammatory niche. It was observed that reduced levels of many of these proteins in the tumors of CDC-EV treated mice compared with controls.

In studying CDC-EVs, a relationship between anti-aging and anti-cancer effects was noted. CDC-EVs from young donors have local and systemic rejuvenating properties. The rejuvenating effects of miR-146 on fibroblasts are associated with inhibition of IL-6 expression, a key mediator of the senescence-associated secretory phenotype. Meanwhile, miR-146 appears to act as a tumor suppressor for many solid and hematological malignancies. Although the mechanism of miR-146-mediated tumor suppression is not fully characterized, EGF-R was identified as a target of this miR. Increased miR-146 and a subsequent decline of EGF-R expression are associated with decreased proliferation, and inhibited invasion and migration of tumor cells in breast, pancreatic and gastric cancer. Mouse miR-146 knockout models strongly support a role for miR-146 as a tumor suppressor for myelo-lymphoid cells. Both IL-6 and EGF-R were negatively modulated by CDC-EVs in the disclosed study, supporting the idea that CDC-EVs may act as a source of miR-146 as one possible anti-oncogenic mechanism. Another miR similarly abundant in human- and rat-CDC-EVs, and significantly higher compared with MSC-EVs, was miR-92, a member of the miR-17-92 cluster, and an important regulator of cancer and aging. HIF-1α was downregulated both with human- and rat-CDC-EVs. Although the evidence suggests that cell type-specific responses are possible in response to miR-17-92, downregulation of miR-92a specifically triggers macrophage infiltration of the tumor stroma, promotes cell migration and decreases survival in breast cancer patients.

Contrary to CDC-EVs, the use of MSC-EVs was associated with greater lung metastasis. MSCs may exert pro-tumorigenic effects by inducing immunosuppression, promoting angiogenesis and/or stimulating epithelial-to-mesenchymal transition. A significant up-regulation of genes implicated in inflammation, and proteins such as cathepsin D, eNOS, EpCAM and ANGPTL4 in MSC-EV vs CDC-EV treated animals. All these proteins have been described as associated with increased growth, invasiveness, angiogenesis and metastasis in different studies. Moreover, miR-222/223 enriched in the MSC-EVs vs CDC-EVs in the disclosed study, was implicated in inducing dormancy and prolonged survival of breast cancer cells together with increased drug resistance.

The various methods and techniques described above provide a number of ways to carry out the invention. Of course, it is to be understood that not necessarily all objectives or advantages described may be achieved in accordance with any particular embodiment described herein. Thus, for example, those skilled in the art will recognize that the methods can be performed in a manner that achieves or optimizes one advantage or group of advantages as taught herein without necessarily achieving other objectives or advantages as may be taught or suggested herein. A variety of advantageous and disadvantageous alternatives are mentioned herein. It is to be understood that some embodiments specifically include one, another, or several advantageous features, while others specifically exclude one, another, or several disadvantageous features, while still others specifically mitigate a present disadvantageous feature by inclusion of one, another, or several advantageous features.

Furthermore, the skilled artisan will recognize the applicability of various features from different embodiments. Similarly, the various elements, features and steps discussed above, as well as other known equivalents for each such element, feature or step, can be mixed and matched by one of ordinary skill in this art to perform methods in accordance with principles described herein. Among the various elements, features, and steps some will be specifically included and others specifically excluded in diverse embodiments.

As disclosed herein, CDCs and CDC-derived EVs and significantly reduce tumor growth in an in vivo cancer model. The role of EVs in cell-cell communication between tumor cells and surrounding cells has been highlighted as relevant to metastasis and tumor growth.

Many variations and alternative elements have been disclosed in embodiments of the invention. Still further variations and alternate elements will be apparent to one of skill in the art. Among these variations, without limitation, are EVs, sources of such EVs, further including techniques and composition and use of solutions used therein, and the particular use of the products created through the teachings of the invention. Various embodiments of the invention can specifically include or exclude any of these variations or elements.

In some embodiments, the terms "a" and "an" and "the" and similar references used in the context of describing a particular embodiment of the invention (especially in the context of certain of the following claims) can be construed to cover both the singular and the plural. The recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g."such as") provided with respect to certain embodiments herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member can be referred to and claimed individually or in any combination with other members of the group or other elements found herein. One or more members of a group can be included in, or deleted from, a group for reasons of convenience and/or patentability. When any such inclusion or deletion occurs, the specification is herein deemed to contain the group as modified thus fulfilling the written description of all Markush groups used in the appended claims.

## Claims

1. Extracellular vesicles derived from cardiosphere-derived cells (CDC-EVs) for use in a method of treating or preventing cancer in a subject in need thereof, the method comprising:
i) administering to the subject a therapeutically effective amount of extracellular vesicles (CDC-EVs); or
ii) administering a composition comprising CDC-EVs to a subject who is susceptible to cancer, wherein the subject possesses one or more genetic mutations that make the subject susceptible to cancer.

2. The CDC-EVs for use in a method according claim 1, part i), wherein said CDC-EVs comprise one or more of, CDC-derived microvesicles (CDC-MVs), CDC-derived exosomes (CDC-XOs), or combinations thereof.

3. The CDC-EVs for use in a method according claim 1, part i), wherein said therapeutically effective amount of CDC-EVs is administered to the subject systemically or locally.

4. The CDC-EVs for use in a method of claim 1, part i), wherein treating cancer in the subject comprises one or more of a reduction in tumor weight, a reduction in tumor vascularization, a reduction in tumor invasion, metastasis, or combinations thereof.

5. The CDC-EVs for use in a method of claim 1, part i), wherein administration of the therapeutically effective amount of CDC-EVs is via two or more injections.

6. The CDC-EVs for use in a method of claim 1, part i), wherein at least two doses of a therapeutically effective amount of CDC EVs are administered.

7. The CDC-EVs for use in a method of claim 6, wherein the at least two doses are administered about 1 to 2 weeks apart from each other.

8. The CDC-EVs for use in a method according to claim 7, wherein the doses are given to the patient via systemic administration, via local administration, or both.

9. The CDC-EVs for use in a method according to any one of claims 7 or 8, wherein said CDC-EVs are allogeneic human CDC-EVs, and the subject is a human.

10. The CDC-EVs for use in a method of claim 1, part i), wherein the CDC-EVs are allogeneic and the subject is a human subject.

11. The CDC-EVs for use in a method of claim 1, part i), wherein the CDC-EVs are provided as a pharmaceutical composition comprising a pharmaceutically acceptable carrier.

12. The CDC-EVs for use in a method of claim 1, part ii), wherein administration is systemic or local.

13. The CDC-EVs for use in a method of any one of claim 1, part ii), and 12, wherein treating cancer in the subject comprises a reduction in tumor weight, a reduction in tumor vascularization, a reduction in tumor invasion and/or metastasis, a decrease in serum lactate dehydrogenase, or combinations thereof.

## Patentansprüche

1. Extrazelluläre Vesikel, die von Kardiosphären-stammenden Zellen (CDC-EVs) abstammen, für die Verwendung in einem Verfahren zur Behandlung oder Prävention von Krebs bei einem Subjekt, das diese erfordert, wobei das Verfahren Folgendes umfasst:
i) Verabreichen einer therapeutisch wirksamen Menge von extrazellulären Vesikeln (CDC-EVs) an das Subjekt; oder
ii) Verabreichen einer Zusammensetzung, die CDC-EVs umfasst, an ein Subjekt, das für Krebs anfällig ist, wobei das Subjekt eine oder mehrere genetische Mutationen besitzt, die das Subjekt für Krebs anfällig machen.

2. CDC-EVs für die Verwendung in einem Verfahren nach Anspruch 1, Teil i), wobei die CDC-EVs eines oder mehrere von CDC-abstammenden Mikrovesikeln (CDC-MVs), CDC-abstammenden Exosomen (CDC-XOs) oder Kombinationen davon umfassen.

3. CDC-EVs für die Verwendung in einem Verfahren nach Anspruch 1, Teil i), wobei die therapeutisch wirksame Menge von CDC-EVs systemisch oder lokal an das Subjekt verabreicht wird.

4. CDC-EVs für die Verwendung in einem Verfahren nach Anspruch 1, Teil i), wobei die Behandlung von Krebs bei dem Subjekt eines oder mehrere einer Reduzierung des Tumorgewichts, einer Reduzierung der Tumorvaskularisierung, einer Reduzierung der Tumorinvasion, Metastasierung oder Kombinationen davon umfasst.

5. CDC-EVs für die Verwendung in einem Verfahren nach Anspruch 1, Teil i), wobei die Verabreichung der therapeutisch wirksamen Menge von CDC-EVs über zwei oder mehr Injektionen erfolgt.

6. CDC-EVs für die Verwendung in einem Verfahren nach Anspruch 1, Teil i), wobei mindestens zwei Dosen einer therapeutisch wirksamen Menge von CDC-EVs verabreicht werden.

7. CDC-EVs für die Verwendung in einem Verfahren nach Anspruch 6, wobei die Verabreichungen der mindestens zwei Dosen etwa 1 bis 2 Wochen getrennt voneinander erfolgen.

8. CDC-EVs für die Verwendung in einem Verfahren nach Anspruch 7, wobei die Dosen über systemische Verabreichung, über lokale Verabreichung oder beide an den Patienten gegeben werden.

9. CDC-EVs für die Verwendung in einem Verfahren nach einem der Ansprüche 7 oder 8, wobei die CDC-EVs allogene menschliche CDC-EVs sind und das Subjekt ein Mensch ist.

10. CDC-EVs für die Verwendung in einem Verfahren nach Anspruch 1, Teil i), wobei die CDC-EVs allogen sind und das Subjekt ein menschliches Subjekt ist.

11. CDC-EVs für die Verwendung in einem Verfahren nach Anspruch 1, Teil i), wobei die CDC-EVs als eine pharmazeutische Zusammensetzung bereitgestellt werden, die einen pharmazeutisch verträglichen Träger umfasst.

12. CDC-EVs für die Verwendung in einem Verfahren nach Anspruch 1, Teil ii), wobei die Verabreichung systemisch oder lokal erfolgt.

13. CDC-EVs für die Verwendung in einem Verfahren nach einem der Ansprüche 1, Teil ii), und 12, wobei die Behandlung von Krebs bei dem Subjekt Folgendes umfasst:
eine Reduzierung des Tumorgewichts, eine Reduzierung der Tumorvaskularisierung,
eine Reduzierung der Tumorinvasion und/oder Metastasierung, eine Abnahme der Serum-Lactatdehydrogenase oder Kombinationen davon.

## Revendications

1. Vésicules extracellulaires dérivées de cellules dérivées de la cardiosphère (CDC-EV), pour une utilisation dans une méthode de traitement ou de prévention d'un cancer chez un sujet en ayant besoin, la méthode comprenant :
i) l'administration au sujet d'une quantité thérapeutiquement efficace de vésicules extracellulaires (CDC-EV) ; ou
ii) l'administration d'une composition comprenant des CDC-EV à un sujet prédisposé au cancer, le sujet possédant une ou plusieurs mutations génétiques qui le rendent prédisposé au cancer.

2. CDC-EV pour une utilisation dans une méthode selon la revendication 1, partie i), lesdites CDC-EV comprenant un(e) ou plusieurs parmi des microvésicules dérivées des CDC (CDC-MV), des exosomes dérivés des CDC (CDC-XO), ou des combinaisons de ceux-ci.

3. CDC-EV pour une utilisation dans une méthode selon la revendication 1, partie i), ladite quantité thérapeutiquement efficace de CDC-EV étant administrée au sujet par voie systémique ou locale.

4. CDC-EV pour une utilisation dans une méthode selon la revendication 1, partie i), le traitement du cancer chez le sujet comprenant une ou plusieurs parmi une réduction du poids des tumeurs, une réduction de la vascularisation des tumeurs, une réduction de l'invasion des tumeurs, des métastases, ou des combinaisons de celles-ci.

5. CDC-EV pour une utilisation dans une méthode selon la revendication 1, partie i), l'administration de la quantité thérapeutiquement efficace de CDC-EV se faisant par deux, ou plus, injections.

6. CDC-EV pour une utilisation dans une méthode selon la revendication 1, partie i), au moins deux doses d'une quantité thérapeutiquement efficace de CDC-EV étant administrées.

7. CDC-EV pour une utilisation dans une méthode selon la revendication 6, les au moins deux doses étant administrées à environ 1 à 2 semaines d'intervalle.

8. CDC-EV pour une utilisation dans une méthode selon la revendication 7, les doses étant administrées au patient via une administration systémique, via une administration locale, ou les deux.

9. CDC-EV pour une utilisation dans une méthode selon l'une quelconque des revendications 7 ou 8, lesdites CDC-EV étant des CDC-EV humaines allogéniques, et le sujet étant un être humain.

10. CDC-EV pour une utilisation dans une méthode selon la revendication 1, partie i), les CDC-EV étant allogéniques et le sujet étant un sujet humain.

11. CDC-EV pour une utilisation dans une méthode selon la revendication 1, partie i), les CDC-EV étant fournies sous forme de composition pharmaceutique comprenant un véhicule pharmaceutiquement acceptable.

12. CDC-EV pour une utilisation dans une méthode selon la revendication 1, partie ii), l'administration étant systémique ou locale.

13. CDC-EV pour une utilisation dans une méthode selon l'une quelconque des revendications 1, partie ii), et 12, le traitement du cancer chez le sujet comprenant une réduction du poids des tumeurs, une réduction de la vascularisation des tumeurs, une réduction de l'invasion des tumeurs et/ou des métastases, une diminution de la lactate déshydrogénase sérique, ou des combinaisons de celles-ci.
